# EUROPEAN PATENT APPLICATION

(11) **EP 2 275 167 A2**
(43) Date of publication of application: **19.01.2011**
(21) Application number: 10179554.0
(22) Date of filing: 03.03.2000
(51) Int. Cl.: A61N 1/00

(54) **Visual prosthesis**

(30) Priority: 24.03.1999 US 125873 P
(62) Divisional of application: 06026156.7
(71) Applicant: Second Sight Medical Products, Inc., Sylmar, CA 91342 (US)
(72) Inventor: Greenberg, Robert, J., Los Angeles, CA 90025 (US); Schulman, Joseph, H., Santa Clarita, CA 91351 (US)
(74) Representative: Carter, Stephen John

(57) **Abstract**

A visual prosthesis comprising: an image receiver for receiving an image and converting said image to a signal; a data processing unit, coupled to said image receiver and processing said signal; a patient's controller within a housing suitable to place external to a human body; a manually operable user interface on said housing, accepting user input and controlling said data processing unit and, thereby, electrode signal parameters; and a plurality of electrodes driven in accordance with said signal and said electrode signal parameters, stimulating visual neurons to create a perception of said image.

## Description

This application claims the benefit of U. S. Provisional Application No. 60/125,873, filed March 24, 1999.

### TECHNICAL FIELD OF INVENTION

The present invention relates to electrical stimulation of the retina to produce artificial images for the brain. It relates to electronic image stabilization techniques based on tracking the movements of the eye. It relates to telemetry in and out of the eye for uses such as remote diagnostics and recording from the retinal surface.

The present invention also relates to electrical stimulation of the retina to produce phosphenes and to produce induced color vision. The present invention relates to hermetically sealed electronic and electrode units which are safe to implant in the eye.

### BACKGROUND

Color perception is part of the fabric of human experience. Homer (c. 1100 b. c.) writes of "the rosy-fingered dawn". Lady Murasaki no Shikibu (c. 1000 a.d.) uses word colors ("purple, yellow shimmer of dresses, blue paper") in the world's first novel. In the early nineteenth century Thomas Young, an English physician, proposed a trichromatic theory of color vision, based on the action of three different retinal receptors. Fifty years later James Clerk Maxwell, the British physicist and Hermann von Helmholtz, the German physiologist, independently showed that all of the colors we see could be made up from three suitable spectral color lights. In 1964 Edward MacNichol and colleagues at Johns Hopkins and George Wald at Harvard measured the absorption by the visual pigments in cones, which are the color receptor cells. Rods are another type of photoreceptor cell in the primate retina. These cells are more sensitive to dimmer light but are not directly involved in color perception. The individual cones have one of three types of visual pigment. The first is most sensitive to short waves, like blue. The second pigment is most sensitive to middle wavelengths, like green. The third pigment is most sensitive to longer wavelengths, like red.

The retina can be thought of a big flower on a stalk where the top of that stalk is bent over so that the back of the flower faces the sun. In place of the sun, think of the external light focused by the lens of the eye onto the back of the flower. The cones and rods cells are on the front of the flower; they get the light that has passed through from the back of the somewhat transparent flower. The photoreceptor nerve cells are connected by synapses to bipolar nerve cells, which are then connected to the ganglion nerve cells. The ganglion nerve cells connect to the optic nerve fibers, which is the "stalk" that carries the information generated in the retina to the brain. Another type of retinal nerve cell, the horizontal cell, facilitates the transfer of information horizontally across bipolar cells. Similarly, another type of cell, the amacrine facilitates the horizontal transfer of information across the ganglion cells. The interactions among the retinal cells can be quite complex. On-center and off-center bipolar cells can be stimulated at the same time by the same cone transmitter release to depolarize and hyperpolarize, respectively. A particular cell's receptive field is that part of the retina, which when stimulated, will result in that cell's stimulation. Thus, most ganglion cells would have a larger receptive field than most bipolar cells. Where the response to the direct light on the center of a ganglion cells receptive field is antagonized by direct light on the surround of its receptive field, the effect is called center-surround antagonism. This phenomenon is important for detecting borders independent of the level of illumination. The existence of this mechanism for sharpening contrast was first suggested by the physicist Ernst Mach in the late 1800's. More detailed theories of color vision incorporate color opponent cells. On the cone level, trichromatic activity of the cone cells occurs. At the bipolar cell level, green-red opponent and blue-yellow opponent processing systems of the center-surround type, occur. For example, a cell with a green responding center would have an annular surround area, which responded in an inhibiting way to red. Similarly there can be red-center responding, green-surround inhibiting response. The other combinations involve blue and yellow in an analogous manner.

It is widely known that Galvani, around 1780, stimulated nerve and muscle response electrically by applying a voltage on a dead frog's nerve. Less well known is that in 1755 LeRoy discharged a Leyden jar, i.e., a capacitor, through the eye of a man who had been blinded by the growth of a cataract. The patient saw "flames passing rapidly downward."

In 1958, Tassicker was issued a patent for a retinal prosthetic utilizing photosensitive material to be implanted subretinally. In the case of damage to retinal photoreceptor cells that affected vision, the idea was to electrically stimulate undamaged retinal cells. The photosensitive material would convert the incoming light into an electrical current, which would stimulate nearby undamaged cells. This would result in some kind of replacement of the vision lost. Tassicker reports an actual trial of his device in a human eye. (U.S. Patent No. 2,760,483).

Subsequently, Michelson (U.S. Patent No.4, 628,933), Chow (U.S. Patent Nos. 5,016,633; 5,397,350; 5,556,423), and De Juan (U.S. Patent No. 5,109,844) all were issued patents relating to a device for stimulating undamaged retinal cells. Chow and Michelson made use of photodiodes and electrodes. The photodiode was excited by incoming photons and produced a current at the electrode.

Normann et al. (U.S. Patent No. 5,215,088) discloses long electrodes 1000 to 1500 microns long designed to be implanted into the brain cortex. These spire-shaped electrodes were formed of a semiconductor material.

Najafi, et al., (U.S. Patent No. 5314458), disclosed an implantable silicon-substrate based microstimulator with an external device which could send power and signal to the implanted unit by RF means. The incoming RF signal could be decoded and the incoming RF power could be rectified and used to run the electronics.

Difficulties can arise if the photoreceptors, the electronics, and the electrodes all tend to be mounted at one place. One issue is the availability of sufficient area to accomodate all of the devices, and another issue is the amount of power dissipation near the sensitive retinal cells. Since these devices are designed to be implanted into the eye, this potential overheating effect is a serious consideration.

Since these devices are implants in the eye, a serious problem is how to hermetically seal these implanted units. Of further concern is the optimal shape for the electrodes and for the insulators, which surround them. In one embodiment there is a definite need that the retinal device and its electrodes conform to the shape of the retinal curvature and at the same time do not damage the retinal cells or membranes.

The length and structure of electrodes must be suitable for application to the retina, which averages about 200 microns in thickness. Based on this average retinal thickness of 200 microns, elongated electrodes in the range of 100 to 500 microns appear to be suitable. These elongated electrodes reach toward the cells to be activated. Being closer to the targeted cell, they require less current to activate it.

In order not to damage the eye tissue there is a need to maintain an average charge neutrality and to avoid introducing toxic or damaging effects from the prosthesis.

A desirable property of a retinal prosthetic system is making it possible for a physician to make adjustments on an on-going basis from outside the eye. One way of doing this would have a physician's control unit, which would enable the physician to make adjustments and monitor the eye condition. An additional advantageous feature would enable the physician to preform these functions at a remote location at a remote location, e.g., from his office. This would allow one physician to remotely monitor a number of patients remotely without the necessity of the patient coming to the office. A patient could be travelling distantly and obtain physician monitoring and control of the retinal color prosthetic parameters.

Another version of the physician's control unit is a hand-held, palm-size unit. This unit will have some, but not all of the functionality of the physician's control unit. It is for the physician to carry on his rounds at the hospital, for example, to check on post-operative retinal-prosthesis implant patients. Its extreme portability makes other situational uses possible, too, as a practical matter.

The patient will want to control certain aspects of the visual image from the retinal prosthesis system, in particular, image brightness. Consequently, a patient controller, performing fewer functions than the physician's controller is included as part of the retinal prosthetic system. It will control, at a minimum, bright image, and it will control this image brightness in a continuous fashion. The image brightness may be increased or decreased by the patient at any time, under normal circumstances.

A system of these components would itself constitute part of a visual prosthetic to form images in real time within the eye of a person with a damaged retina. In the process of giving back sight to those who are unable to see, it would be advantageous to supply artificial colors in this process of reconstructing sight so that the patient would be able to enjoy a much fuller version of the visual world.

In dealing with externally mounted or externally placed means for capturing image and transmitting it by electronic means or other into the eye, one must deal with the problem of stabilization of the image. For example, a head-mounted camera would not follow the eye movement. It is desirable to track the eye movements relative to the head and use this as a method or approach to solving the image stabilization problem.

By having a method and apparatus for the physician and the technician to initially set up and measure the internal activities and adjust these, the patient's needs can be better accommodated. The opportunity exists to measure internal activity and to allow the physician, using his judgement, to adjust settings and controls on the electrodes. Even the individual electrodes would be adjusted by way of the electronics controlling them. By having this done remotely, by remote means either by telephone or by the Internet or other such, it is clear that a physician would have the capability to intervene and make adjustment as necessary in a convenient and inexpensive fashion, to serve many patients.

### SUMMARY OF INVENTION

The objective of the current invention is to restore color vision, in whole or in part, by electrically stimulating undamaged retinal cells, which remain in patients with lost or degraded visual function arising, for example, from Retinitis Pigmentosa or Age-Related Macular Degeneration. This invention is directed toward patients who have been blinded by degeneration of photoreceptors; but who have sufficient bipolar cells, or other cells acting similarly, to permit electrical stimulation.

There are three main functional parts to this invention. One is external to the eye. The second part is internal to the eye. The third part is the communication circuitry for communicating between those two parts. Structurally there are two parts. One part is external to the eye and the other part is implanted internal to the eye. Each of these structural parts contains two way communication circuitry for communication between the internal and external parts.

The structural external part is composed of a number of subsystems. These subsystems include an external imager, an eye-motion compensation system, a head motion compensation system, a video data processing unit, a patient's controller, a physician's local controller, a physician's remote controller, and a telemetry unit. The imager is a video camera such as a CCD or CMOS video camera. It gathers an image approximating what the eyes would be seeing if they were functional.

The imager sends an image in the form of electrical signals to the video data processing unit. In one aspect, this unit formats a grid-like or pixel-like pattern that is then ultimately sent to electronic circuitry (part of the internal part) within the eye, which drives the electrodes. These electrodes are inside the eye. They replicate the incoming pattern in a useable form for stimulation of the retina so as to reproduce a facsimile of the external scene. In an other aspect of this invention other formats other than a grid-like or pixel like pattern are used, for example a line by line scan in some order, or a random but known order, point-by-point scan. Almost any one-to-one mapping between the acquired image and the electrode array is suitable, as long as the brain interprets the image correctly.

The imager acquires color information. The color data is processed in the video data processing unit. The video data processing unit consists of microprocessor CPU's and associated processing chips including high-speed data signal processing (DSP) chips.

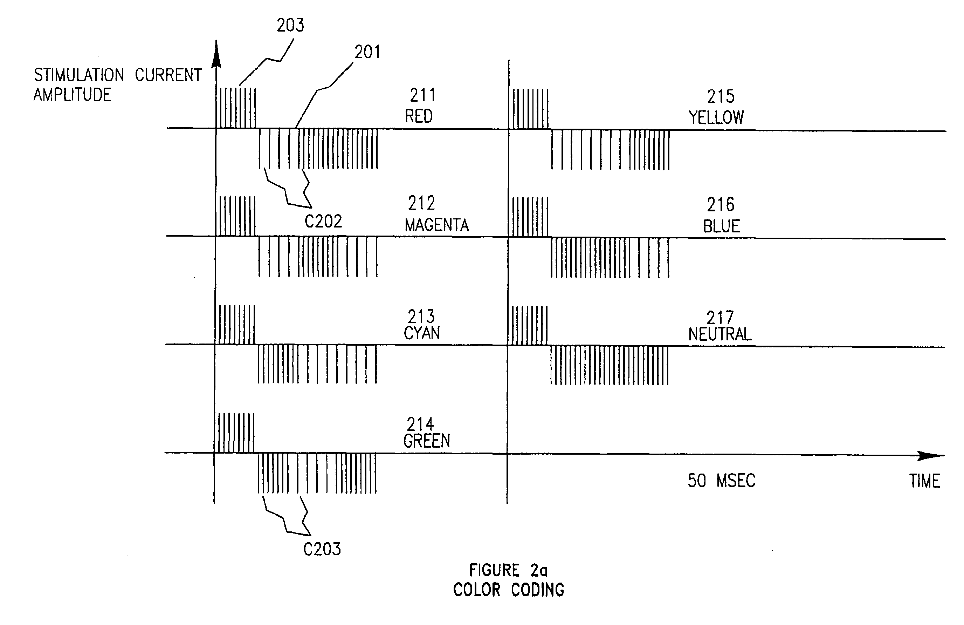

In one aspect, the color information is encoded by time sequences of pulses separated by varying amounts of time; and, the pulse duration may be different for various pulses. The basis for the color encoding is the individual color code reference (Figure 2a). The electrodes stimulate the target cells so as to create a color image for the patient, corresponding to the original image as seen by the video camera, or other imaging means.

Color information, in an alternative aspect, is sent from the video data processing unit to the electrode array, where each electrode has been determined to stimulate preferentially one of the bipolar cell types, namely, red-center green-surround, green-center-red-surround, blue-center-yellow-surround, or yellow-center-blue-surround.

An eye-motion compensation system is an aspect of this invention. The eye tracker is based on detection of eye motion from the corneal reflex or from implanted coils of wire, or, more generally, insulated conductive coils, on the eye or from the measurement of electrical activity of extra-ocular muscles. Communication is provided between the eye tracker and the video data processing unit by electromagnetic or acoustical telemetry. In one embodiment of the invention, electromagnetic-based telemetry may be used. The results of detecting the eye movement are transmitted to a video data processing unit, together with the information from the camera means. Another aspect of the invention utilizes a head motion sensor and head motion compensation system. The video data processing unit can incorporate the data of the motion of the eye as well as that of the head to further adjust the image electronically so as to account for eye motion and head motion.

The internal structural part, which is implanted internally within the eye, is also composed of a number of subsystems. These can be categorized as electronic circuits and electrode arrays, and communication subsystems, which may include electronic circuits. The circuits, the communication subsystems, and the arrays can be hermetically sealed and they can be attached one to the other by insulated wires. The electrode arrays and the electronic circuits can be on one substrate, or they may be on separate substrates joined by an insulated wire or by a plurality of insulated wires. This is similarly the case for a communication subsystem.

A plurality of predominately electronic substrate units and a plurality of predominately electrode units may be implanted or located within the eye as desired or as necessary. The electrodes are designed so that they and the electrode insulation conform to the retinal curvature. The variety of electrode arrays include recessed electrodes so that the electrode array surface coming in contact with the retinal membrane or with the retinal cells is the non-metallic, more inert insulator.

Another aspect of the invention is the elongated electrode, which is designed to stimulate deeper retinal cells by penetrating into the retina by virtue of the length of its electrodes. A plurality of electrodes is used. The elongated electrodes are of lengths from 100 microns to 500 microns. With these lengths, the electrode tips can reach through those retinal cells not of interest but closer to the target stimulation cells, the bipolar cells. The number of electrodes may range from 100 on up to 10,000 or more. With the development of electrode fabrication technology, the number of electrodes might rage up to one million or more.

Another aspect of the invention uses a plurality of capacitive electrodes to stimulate the retina, in place of non-capacitive electrodes. Another aspect of the invention is the use of a neurotrophic factor, for example, Nerve Growth Factor, applied to the electrodes, or to the vicinity of the electrodes, to aid in attracting target nerves and other nerves to grow toward the electrodes.

Hermetic sealing is accomplished by coating the object to be sealed with a substance selected from the group consisting of silicon carbide, diamond-like coating, silicon nitride and silicon oxide in combination, titanium oxide, tantalum oxide, aluminum nitride, aluminum oxide, zirconium oxide. This hermetic sealing aspect of the invention provides an advantageous alternative to glass coverings for hermetic seals, being less likely to become damaged.

Another feature of one aspect of the structural internal-to-the-eye subsystems is that the electronics receive and transmit information in coded or pulse form via electromagnetic waves. In the case where electromagnetic waves are used, the internal-to-the-eye-implanted electronics can rectify the RF, or electromagnetic wave, current and decode it. The power being sent in through the receiving coil is extracted and used to drive the electronics. In some instances, the implanted electronics acquire data from the electrode units to transmit out to the video data processing unit.

In another aspect the information coding is done with ultrasonic sound. An ultrasonic transducer replaces the electromagnetic wave receiving coil inside the eye. An ultrasonic transducer replaces the coil outside the eye for the ultrasonic case. By piezoelectric effects, the sound vibration is turned into electrical current, and energy extracted therefrom.

In another aspect of the invention, information is encoded by modulating light. For the light modulation case, a light emitting diode (LED) or laser diode or other light generator, capable of being modulated, acts as the information transmitter. Information is transferred serially by modulating the light beam, and energy is extracted from the light signal after it is converted to electricity. A photo-detector, such as a photodiode, which turns the modulated light signal into a modulated electrical signal, is used as a receiver.

Another aspect of the structural internal-to-the-eye subsystems of this invention is that the predominately electrode array substrate unit and the predominately electronic substrate unit, which are joined by insulated wires, can be placed near each other or in different positions. For example, the electrode array substrate unit can be placed subretinally and the electronic substrate unit placed epiretinally. On a further aspect of this invention, the electronic substrate unit can be placed distant from the retina so as to avoid generating additional heat or decreasing the amount of heat generated near the retinal nerve system. For example, the receiving and processing circuitry could be placed in the vicinity of the pars plana. In the case where the electronics and the electrodes are on the same substrate chip, two of these chips can be placed with the retina between them, one chip subretinal and the other chip epiretinal, such that the electrodes on each may be aligned. Two or more guide pins with corresponding guide hole or holes on the mating chip accomplish the alignment. Alternatively, two or more tiny magnets on each chip, each magnet of the correct corresponding polarity, may similarly align the sub- and epiretinal electrode bearing chips. Alternatively, corresponding parts which mate together on the two different chips and which in a fully mated position hold each other in a locked or "snap-together" relative position.

Now as an element of the external-to-the-eye structural part of the invention.

there is a provision for a physician's hand-held test unit and a physician's local or remote office unit or both for control of parameters such as amplitudes, pulse widths, frequencies, and patterns of electrical stimulation.

The physician's hand-held test unit can be used to set up or evaluate and test the implant during or soon after implantation at the patient's bedside. It has, essentially, the capability of receiving what signals come out of the eye and having the ability to send information in to the retinal implant electronic chip. For example, it can adjust the amplitudes on each electrode, one at a time, or in groups. The hand-held unit is primarily used to initially set up and make a determination of the success of the retinal prosthesis.

The physician's local office unit, which may act as a set-up unit as well as a test unit, acts directly through the video data processing unit. The remote physician's office unit would act over the telephone lines directly or through the Internet or a local or wide area network. The office units, local and remote, are essentially the same, with the exception that the physician's remote office unit has the additional communications capability to operate from a location remote from the patient. It may evaluate data being sent out by the internal unit of the eye, and it may send in information. Adjustments to the retinal color prosthesis may be done remotely so that a physician could handle a multiple number of units without leaving his office. Consequently this approach minimizes the costs of initial and subsequent adjustments.

### BRIEF DESCRIPTION OF THE DRAWINGS

The above and other features and advantages of the invention will be more apparent from the following detailed description wherein:
Figure 1a shows the general structural aspects of the retina color prosthesis system;
Figure 1b shows the retina color prosthesis system with a structural part internal (to the eye), with an external part with subsystems for eye-motion feedback to enable maintaining a stable image presentation, and with a subsystems for communicating between the internal and external parts, and other structural subsystems;
Figure 1c shows an embodiment of the retina color prosthesis system which is, in part, worn in eyeglass fashion; Figure 1d shows the system in Figure 1c in side view;
Figure 2a shows an embodiment of the color I coding schemata for the stimulation of the sensation of color;
Figure 2b represents an embodiment of the color I conveying method where a "large" electrode stimulates many bipolar cells with the color coding schemata of Figure 2a;
Figure 2c represents an embodiment of the color II conveying method where an individual electrode stimulates a single type of bipolar cell;
Figure 3a represents an embodiment of the telemetry unit including an external coil, an internal (to the eye) coil, and an internal electronic chip;
Figure 3b represents an embodiment of the telemetry unit including an external coil, an internal (to the eye) coil, an external electronic chip, a dual coil transfer unit, and an internal electrode array;
Figure 3c shows and acoustic energy and information transfer system;
Figure 3d shows a light energy and information transfer system;
Figure 4 represents an embodiment of the external telemetry unit;
Figure 5 shows an embodiment of an internal telemetry circuit and electrode array switcher;
Figure 6a shows a monopolar electrode arrangement and illustrates a set of round electrodes on a substrate material;
Figure 6b shows a bipolar electrode arrangement;
Figure 6c shows a multipolar electrode arrangement;
Figure 7 shows the corresponding indifferent electrode for monopolar electrodes;
Figure 8a depicts the location of an epiretinal electrode array located inside the eye in the vitreous humor located above the retina, toward the lens capsule and the aqueous humor;
Figure 8b shows recessed epiretinal electrodes where the electrically conducting electrodes are contained within the electrical insulation material; a silicon chip acts as a substrate; and the electrode insulator device is shaped so as to contact the retina in a conformal manner;
Figure 8c is a rendering of an elongated epiretinal electrode array with the electrodes shown as pointed electrical conductors, embedded in an electrical insulator, where an pointed electrodes contact the retina in a conformal manner, however, elongated into the retina;
Figure 9a shows the location of a subretinal electrode array below the retina, away from the lens capsule and the aqueous humor. The retina separates the subretinal electrode array from the vitreous humor;
Figure 9b illustrates the subretinal electrode array with pointed elongated electrode, the insulator, and the silicon chip substrate where the subretinal electrode array is in conformal contact with the retina with the electrodes elongated to some depth;
Figure 10a shows a iridium electrode that comprises a iridium slug, an insulator, and a device substrate where this embodiment shows the iridium slug electrode flush with the extent of the insulator;
Figure 10b indicates an embodiment similar to that shown in Figure 10/12a, however, the iridium slug is recessed from the insulator along its sides, but with its top flush with the insulator;
Figure 10c shows an embodiment with the iridium slug as in Figure 10/12b;
   however, the top of the iridium slug is recessed below the level of the insulator;
Figure 10d indicates an embodiment with the iridium slug coming to a point and insulation along its sides, as well as a being within the overall insulation structure;

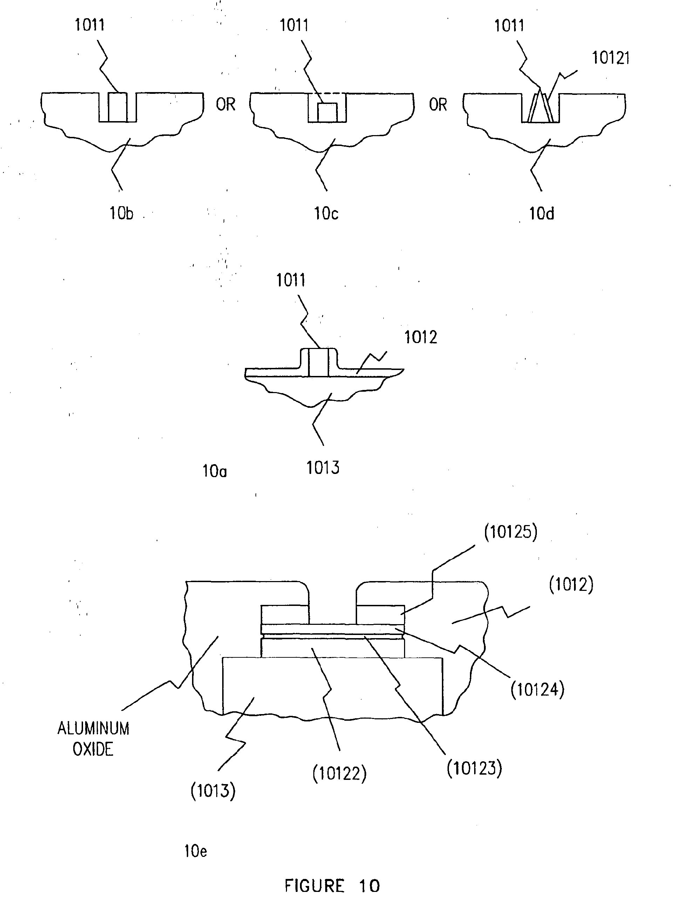

Figure 10e indicates an embodiment of a method for fabricating and the fabricated iridium electrode where on a substrate of silicon an aluminum pad is deposited; on the pad the conductive adhesive is laid and platinum or iridium foil is attached thereby; a titanium ring is placed, sputtered, plated, ion implanted, ion beam assisted deposited (IBAD) or otherwise attached to the platinum or iridium foil; silicon carbide, diamond-like coating, silicon nitride and silicon oxide in combination, titanium oxide, tantalum oxide, aluminum nitride, aluminum oxide or zirconium oxide or other insulator will adhere better to the titanium while it would not adhere as well to the platinum or iridium foil;
Figure 11a depicts a preferred electrode where it is formed on a silicon substrate and makes use of an aluminum pad, a metal foil such as platinum or iridium, conductive adhesive, a titanium ring, aluminum or zirconium oxide, an aluminum layer, and a mask;
Figure 11b shows an elongated electrode formed on the structure of Figure 11 a with platinum electroplated onto the metal foil, the mask removed and insulation applied over the platinum electrode;
Figure 11c shows a variation of a form of the elongated electrode wherein the electrode thinner and more recessed from the well sides;
Figure 11d shows a variation of a form of the elongated electrode, the electrode squatter but recessed from the well sides;
Figure 11e shows a variation of a form of the elongated electrode, the electrode a mushroom shape with the sides of its tower recessed from the well sides and its mushroom top above the oxide insulating material;
Figure 12a shows the coil attachment to two different conducting pads at an electrode nodes;
Figure 12b shows the coil attachment to two different conducting pads at an electrode node, together with two separate insulated conducting electrical pathways such as wires, each attached at two different electrode node sites on two different substrates;
Figure 12c shows an arrangement similar to that seen in Figure 12/16d, with the difference that the different substrates are very close with a non-conducting adhesive between them and an insulator such as aluminum or zirconium oxide forms a connection coating over the two substrates, in part;
Figure 12d depicts an arrangement similar to that seen in Figure 12/16c;
   however, the connecting wires are replaced by an externally placed aluminum conductive trace;
Figure 13 shows a hermetically sealed flip-chip in a ceramic or glass case with solder ball connections to hermetically sealed glass frit and metal leads;
Figure 14 shows a hermetically sealed electronic chip as in Figure 18 with the addition of biocompatible leads to pads on a remotely located electrode substrate;
Figure 15 shows discrete capacitors on the electrode-opposite side of an electrode substrate;
Figure 16a shows an electrode-electronics retinal implant placed with the electrode half implanted beneath the retina, subretinally, while the electronics half projects above the retina, epiretinally;
Figure 16b shows another form of sub- and epi- retinal implantation wherein half of the electrode implant is epiretinal and half is subretinal;
Figure 16c shows the electrode parts are lined up by alignment pins or by very small magnets;
Figure 16d shows the electrode part lined up by template shapes which may snap together to hold the parts in a fixed relationship to each other;
Figure 17a shows the main screen of the physician's (local) controller (and programmer);
Figure 17b illustrates the pixel selection of the processing algorithm with the averaging of eight surrounding pixels chosen as one element of the processing;
Figure 17c represents an electrode scanning sequence, in this case the predefined sequence, A;
Figure 17d shows electrode parameters, here for electrode B, including current amplitudes and waveform timelines;
Figure 17e illustrates the screen for choosing the global electrode configuration, monopolar, bipolar, or multipolar;
Figure 17f renders a screen showing the definition of bipolar pairs (of electrodes);
Figure 17g shows the definition of the multipole arrangements;
Figure 18a illustrates the main menu screen for the palm-sized test unit;
Figure 18b shows a result of pressing on the stimulate bar of the main menu screen, where upon pressing the start button the amplitudes A1 and A2 are stimulated for times t1, t2, t3, and t4, until the stop button is pressed;
Figures 18c exhibits a recording screen that shows the retinal recording of the post-stimulus and the electrode impedance;
Figure 19 shows the physician's remote controller that has the same functionality inside as the physician's controller but with the addition of communication means such as telemetry or telephone modem;
Figure 20 shows the patient's controller unit;

### DESCRIPTION OF THE PREFERRED EMBODIMENTS

The following description is of the best mode presently contemplated for carrying out the invention. This description is not to be taken in a limiting sense, but is merely made for the purpose of describing the general principles of the invention. The scope of the invention should be determined with reference to the claims.

### Objective

The objective of the embodiments of the current invention is a retinal color prosthesis to restore color vision, in whole or in part, by electrically stimulating undamaged retinal cells, which remain in patients with, lost or degraded visual function. Embodiments of this retinal color prosthesis invention are directed toward helping patients who have been blinded by degeneration of photoreceptors and other cells; but who have sufficient bipolar cells and the like to permit the perception of color vision by electric stimulation. By color vision, it is meant to include black, gray, and white among the term color.

### General description

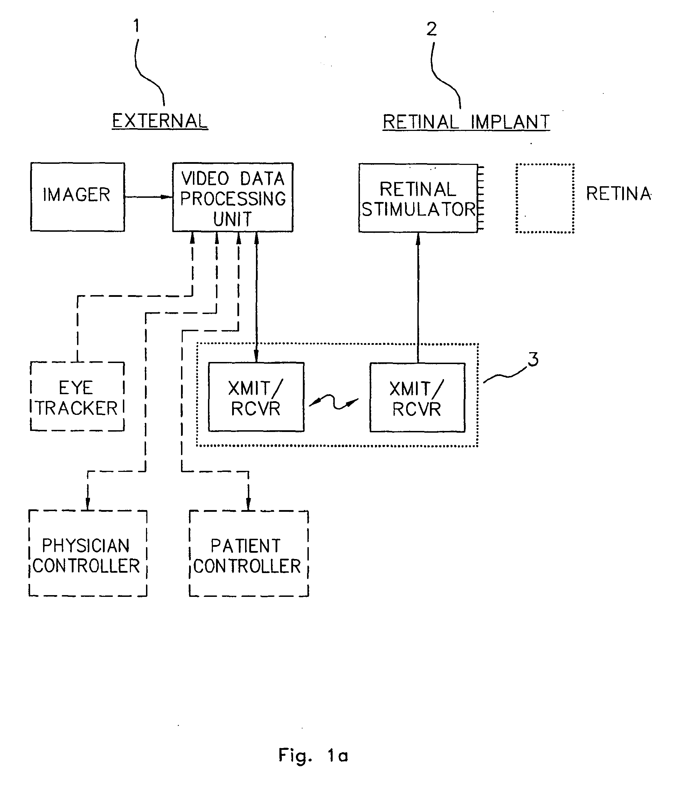

Functionally, there are three main parts to an embodiment of this retinal color prosthesis invention. See Figure 1a. Figure 1a is oriented toward showing the main structural parts and subsystems, with a dotted enclosure to indication a functional intercommunications aspect. The first part of the embodiment is external (1) to the eye. The second part is implanted internal (2) to the eye. The third part is means for communication between those two parts (3). Structurally there are two parts. One part is external (1) to the eye and the other part (2) is implanted within the eye. Each of these structural parts contains two way communication circuitry for communication (3) between the internal (2) and external (1) parts.

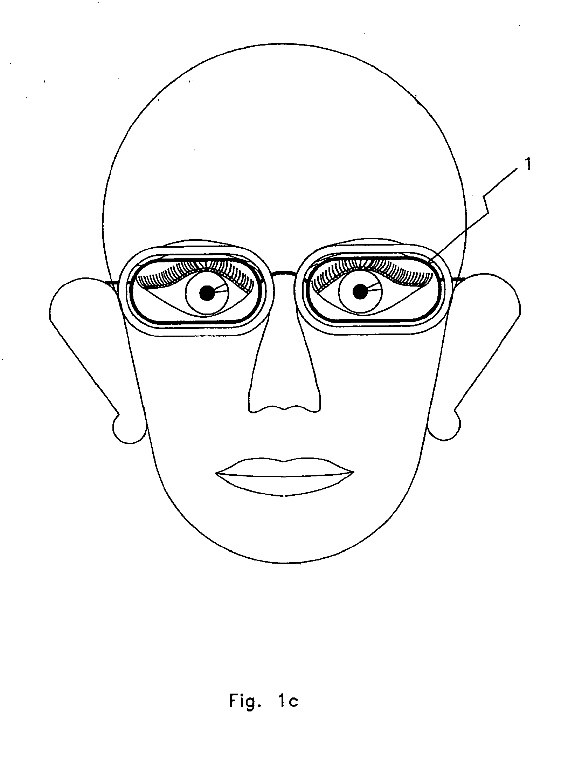

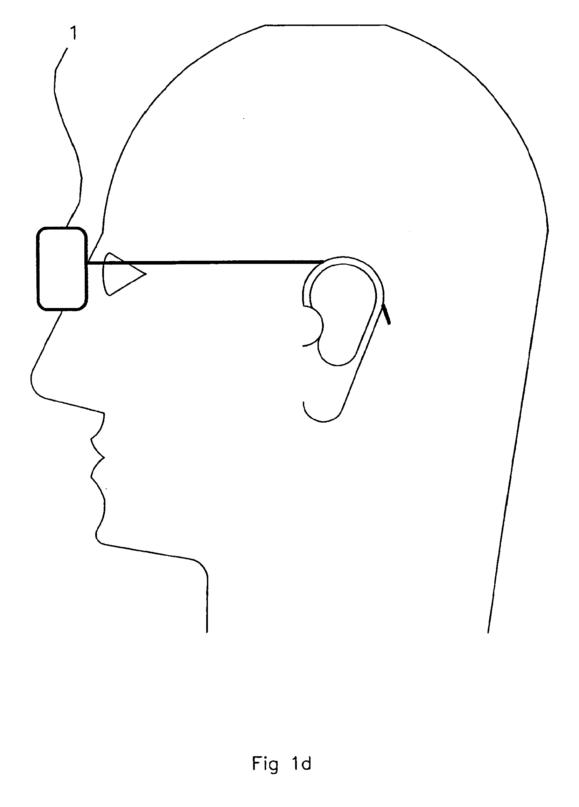

The external part of the retinal color prosthesis is carried by the patient. Typically, the external part including imager, video data processing unit, eye-tracker, and transmitter/receiver are worn as an eyeglass-like unit. Typical of this embodiment, a front view of one aspect of the structural external part (1) of the color retinal prosthesis is shown in Figure 1c and a side view is shown in Figure 1d, (1). In addition, there are two other units, which may be plugged into the external unit; when this is done they act as part of the external unit. The physician's control unit is not normally plugged into the external part worn by the patient, except when the physician is conducting an examination and adjustment of the retinal color prosthetic. The patient's controller may or may not be normally plugged in. When the patient's controller is plugged in, it can also receive signals from a remote physician's controller, which then acts in the same way as the plug-in physician's controller.

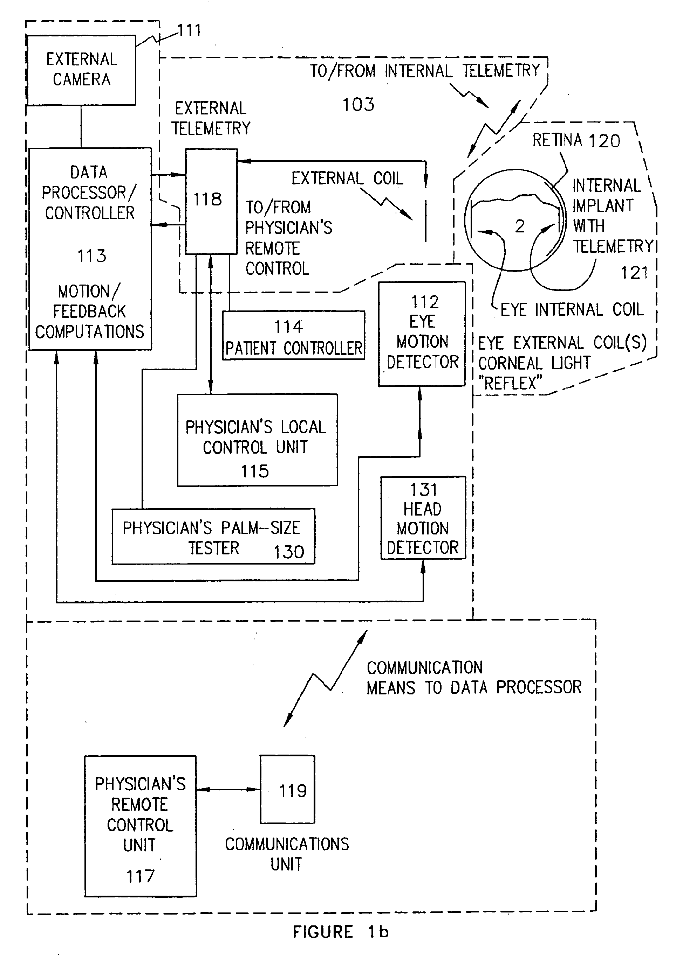

Examining further the embodiment of the subsystems of the external part, see Figure 1b. These include an external color imager (111), an eye-motion compensation system (112), a head-motion compensation system (131), a processing unit (113), a patient's controller (114), a physician's local controller (115), a physicians hand-held palm-size pocket-size unit (116), a physician's remote controller (117), and a telemetry means (118). The color imager is a color video camera such as a CCD or CMOS video camera. It gathers an image approximating what the eyes would be seeing if they were functional.

An external imager (111) sends an image in the form of electrical signals to the video data processing unit (113). The video data processing unit consists of microprocessor CPU's and associated processing chips including high-speed data signal processing (DSP) chips. This unit can format a grid-like or pixel-like pattern that is sent to the electrodes by way of the telemetry communication subsystems (118, 121). See Figure 1b. In this embodiment of the retinal color prosthesis (Fig. 1b, (121)), these electrodes are incorporated in the internal-to-the eye implanted part.

These electrodes, which are part of the internal implant (121), together with the telemetry circuitry (121) are inside the eye. With other internally implanted electronic circuitry (121), they cooperate with the electrodes so as to replicate the incoming pattern, in a useable form, for stimulation of the retina so as to reproduce a facsimile perception of the external scene. The eye-motion (112) and head-motion (131) detectors supply information to the video data processing unit (113) to shift the image presented to the retina (120).

There are three preferred embodiments for stimulating the retina via the electrodes to convey the perception of color. Color information is acquired by the imaging means (111). The color data is processed in the video data processing unit (113).

### First preferred color mode

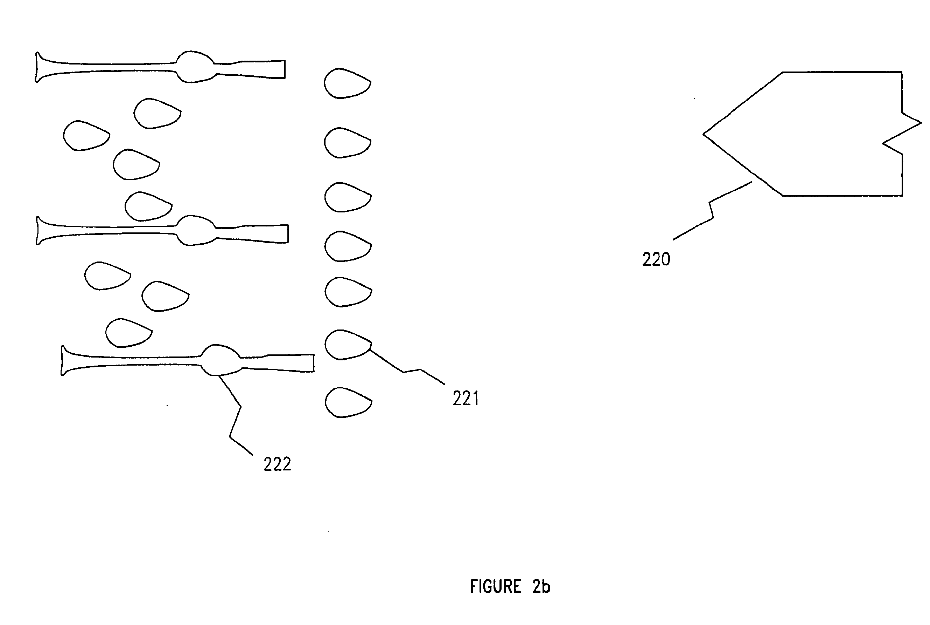

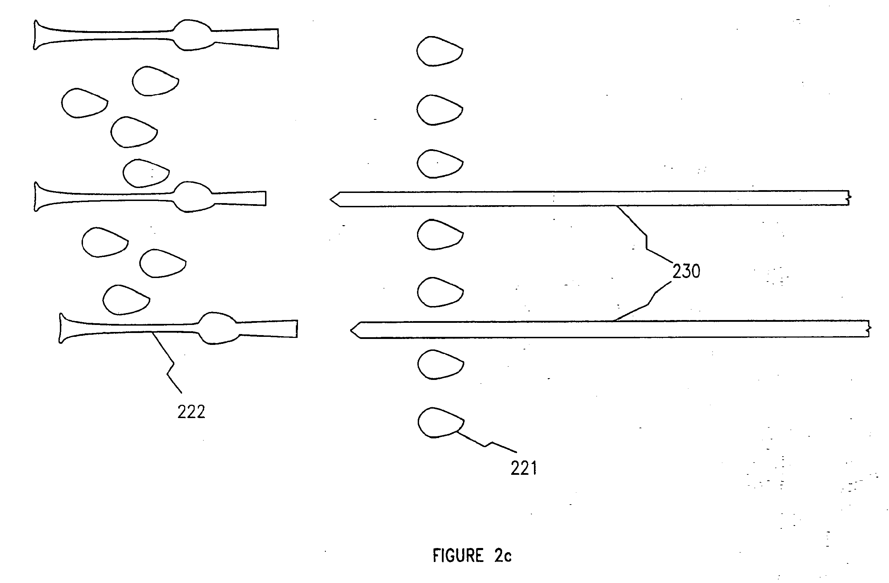

Color information (See Figure 2a), in the first preferred embodiment, is encoded by time sequences of pulses (201) separated by varying amounts of time (202), and also with the pulse duration being varied in time (203). The basis for the color encoding is the individual color code reference (211 through 218). The electrodes stimulate the target cells so as to create a color image for the patient, corresponding to the original image as seen by the video camera, or other imaging means. Using temporal coding of electrical stimuli placed (cf. Figure 2b, 220, Figure 2c, 230) on or near the retina (Figure 2b and Figure 2c, 221, 222) the perception of color can be created in patients blinded by outer retinal degeneration. By sending different temporal coding schemes to different electrodes, an image composed of more than one color can be produced. Figure 2 shows one stimulation protocol. Cathodic stimuli (202) are below the zero plane (220) and anodic stimuli (203) are above. All the stimulus rates are either "fast" (203) or "slow" (202) except for green (214), which includes an intermediate stimulus rate (204). The temporal codes for the other colors are shown as Red (211), as Magenta (212), as Cyan (213), as Yellow (215), as Blue (216), as Neutral (218). This preferred embodiment is directed toward electrodes that are less densely packed in proximity to the retinal cells.

### Second preferred color mode

Color information, in a second preferred embodiment, is sent from the video data processing unit to the electrode array, where each electrode has been determined by test to stimulate one of a bipolar type: red-center green-surround, green-center-red-surround, blue-center-yellow-surround, or yellow-center-blue-surround. In this embodiment, electrodes which are small enough to interact with a single cell, or at most, a few cells. These electrodes are placed in the vicinity of individual bipolar cells, which react to a stimulus with nerve pulse rates and nerve pulse structure (i.e., pulse duration and pulse amplitude). Some of the bipolar cells, when electrically, or otherwise, stimulated, will send red-green signals to the brain. Others will send yellow-blue signals. This refers to the operation of the normal retina. In the normal retina, red or green color photoreceptors (cone cells) send nerve pulses to the red-green bipolar cell which then pass some form of this information up to the ganglion cells and then up to the visual cortex of the brain. With small electrodes individual bipolar cells can be excited in a spatial, or planar, pattern. Small electrodes are those with tip from 0.1 µm to 15 µm, and which individual electrodes are spaced apart from a range 8 µm to 24 µm, so as to approximate a one-to-one correspondence with the bipolar cells. The second preferred embodiment is oriented toward a more densely packed set of electrodes.

### Third preferred color mode

A third preferred mode is a combination of the first and of the second preferred modes such that a broader area coverage of the color information encoded by time sequences of pulses, of varying widths and separations and with relatively fewer electrodes is combined with a higher density of electrodes, addressing more the individual bipolar cells.

### First order and higher effects

Regardless of a particular theory of color vision, the impinging of colored light on the normal cones, and possibly rods, give rise in some fashion to the perception of color, i.e., multi-spectral vision. In the time-pulse coding color method, above, the absence of all, or sufficient, numbers of working cones (and rods) suggests a generalization of the particular time-pulse color encoding method. The generalization is based on the known, or partly known, neuron conduction pathways in the retina. The cone cells, for example, signal to bipolar cells, which in turn signal the ganglion cells. The original spatial-temporal-color (including black, white) schema for conveying color information as the cone is struck by particular wavelength photons is then transformed to a patterned signal firing of the next cellular level, say the bipolar cells, the cones are absent or don't function. Thus, this second level of patterned signal firing is what one wishes to supply to induce the perception of color vision.

The secondary layer of patterned firing may be close to the necessary primary pattern, in which case the secondary pattern **(S)** may be represented as **P *(1 + ε).** The * indicates matrix multiplication. **P** is the primary pattern, represented as a matrix **P** = [ plₗ pₗⱼ ] [Pₖₗ pₖⱼ] where **P** represents the light signals of a particular spatial-temporal pattern, e.g., flicker signals for green. The output from the first cell layer, say the cones, is then **S**, the secondary pattern. This represents the output from the bipolar layer in response to the input from the first (cone) layer. If **S= P*(1+ε),** where (represents a (vector) small deviation applied to the vector 1, then **S** is represented by **P** to the lowest order, and by **P*(1+ε)** to the next order. Thus the response may be seen as a zero order effect and a first order linear effect. Additional terms in the functional relationship are included to completely define the functional relationship. If **S** is some non-linear function of **P,** finding **S** by starting with **P** requires more terms then the linear case to define the bulk of the functional relationship. However, regardless of the details of one color vision theory or another, on physiological grounds **S** is some function of **P.** As in the case of fitting individual patients with lenses for their glasses, variations of parameters are expected in fitting each patient to a particular temporal coding of electrical stimuli.

### Scaling data from photoreceptors to bipolar cells

As cited above, Greenberg (1998) indicates that electrical and photic stimulation of the normal retina operate via similar mechanisms. Thus, even though electrical stimulation of a retina damaged by outer retinal degeneration is different from the electrical stimulation of a normal retina the temporal relationships are expected to be analogous.

To explain this it is noted that electrical stimulation of the normal retinal is accomplished by stimulating the photoreceptor cells (including the color cells activated differentially according to the color of light impinging on them). For the outer retinal degeneration, it is precisely these photoreceptor cells which are missing. Therefore, the electrical stimulation in this case proceeds by way of the cells next up the ladder toward the optic nerve, namely, the bipolar cells.

The time constant for stimulating photoreceptor is about 20 milliseconds. Thus the electrical pulse duration would need to be at least 20 milliseconds. The time constant for stimulating bipolar cells is around 9 seconds. These time constants are much longer than for the ganglion cells (about 1 millisecond). The ganglion cells are another layer of retinal cells closer to the optic nerve. The actual details of the behavior of the different cell types of the retina are quite complicated including the different relationships for current threshold versus stimulus duration (cf. Greenberg, 1998). One may, however, summarize an apparent resonant response of the cells based on their time constants corresponding to the actual pulse stimulus duration.

In Figure 2, which is extrapolated from external-to-the-eye electrical stimulation data of Young (1977) and from light stimulation data of Festinger, Allyn, and White (1971), would be applicable to the photoreceptor cells. One may scale the data down based on the ratio of the photoreceptor time constant (about 20 milliseconds) to that of the bipolar cells (about 9 milliseconds). Consequently, 50 milliseconds on the time scale in Figure 2 now corresponds to 25 milliseconds. Advantageously, stimulation rates and duration of pulses, as well as pulse widths may be chosen which apply to the electrode stimulation of the bipolar cells of the retina.

### Eye movement/ head motion compensation

In a preferred embodiment, an external imager such as a color CCD or color CMOS video camera (111) and a video data processing unit (113), with an external telemetry unit (118) present data to the internal eye-implant part. Another aspect of the preferred embodiment is a method and apparatus for tracking eye movement (112) and using that information to shift (113) the image presented to the retina. Another aspect of the preferred embodiment utilizes a head motion sensor (131) and a head motion compensation system (131, 113). The video data processing unit incorporates the data of the motion of the eye as well as that of the head to further adjust the image electronically so as to account for eye motion and head motion. Thus electronic image compensation, stabilization and adjustment are provided by the eye and head movement compensation subsystems of the external part of the retinal color prosthesis.

### Logarithmic encoding of light

In one aspect of an embodiment (Figure 1b), light amplitude is recorded by the external imager (111). The video data processing unit using a logarithmic encoding scheme (113) to convert the incoming light amplitudes into the logarithmic electrical signals of these amplitudes (113). These electrical signals are then passed on by telemetry (118), (121), to the internal implant (121) which results in the retinal cells (120) being stimulated via the implanted electrodes (121), in this embodiment as part of the internal implant (121). Encoding is done outside the eye, but maybe done internal to the eye, with a sufficient internal computational capability.

### Energy and signal transmission

Coils

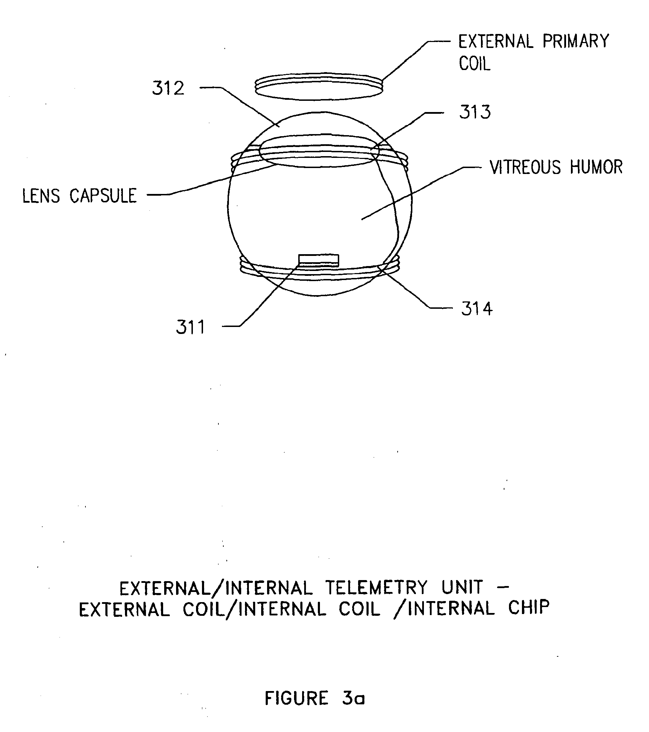

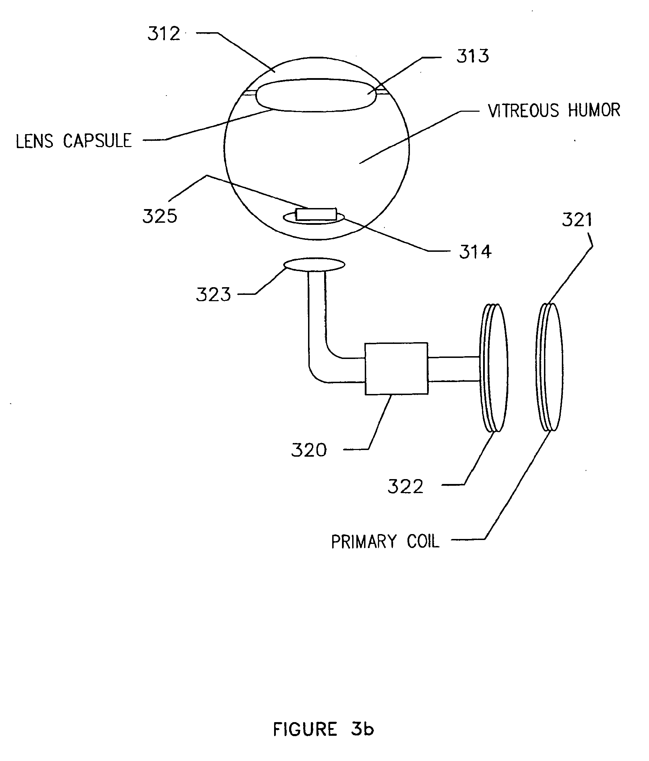

The retinal prosthesis system contains a color imager (Figure 1b, 111) such as a color CCD or CMOS video camera. The imaging output data is typically processed (113) into a pixel-based format compatible with the resolution of the implanted system. This processed data (113) is then associated with corresponding electrodes and amplitude and pulse-width and frequency information is sent by telemetry (118) into the internal unit coils, (311), (313), (314) (see Figure 3a). Electromagnetic energy, is transferred into and out from an electronic component (311) located internally in the eye (312), using two insulated coils, both located under the conjunctiva of the eye with one free end of one coil (313) joined to one free end of the second coil (314), the second free end of said one coil joined to the second free end of said second coil. The second coil (314) is located in proximity to a coil (311) which is a part of said internally located electronic component, or, directly to said internally located electronic component (311). The larger coil is positioned near the lens of the eye. The larger coil is fastened in place in its position near the lens of the eye, for example, by suturing. Figure 3b represents an embodiment of the telemetry unit temporally located near the eye, including an external temporal coil (321), an internal (to the eye) coil (312), an external-to-the-eye electronic chip (320), dual coil transfer units (314, 323), (321,322) and an internal-to-the-eye electrode array (325). The advantage of locating the external electronics in the fatty tissue behind the eye is that there is a reasonable amount of space there for the electronics and in that position it appears not to interfere with the motion of the eye.

### Ultrasonic sound

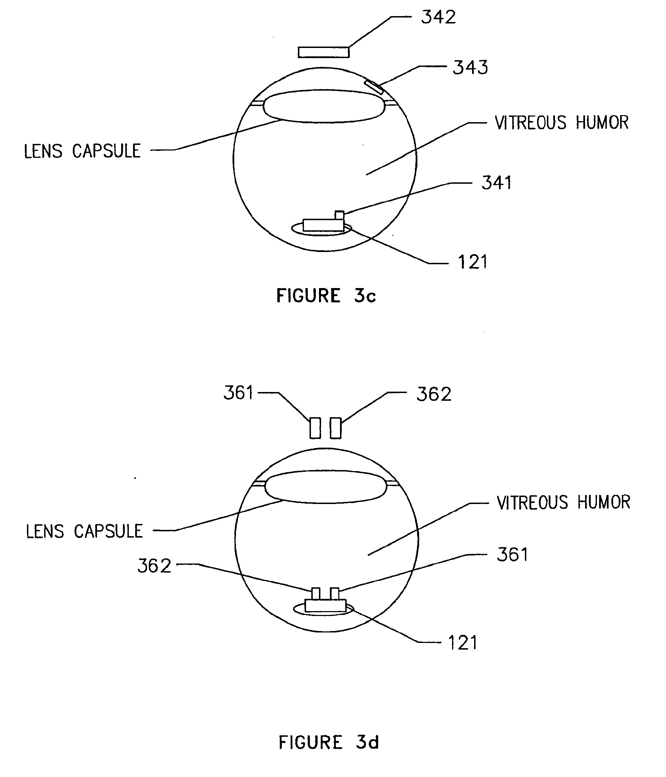

In another aspect the information coding is done with ultrasonic sound and in a third aspect information is encoded by modulating light. An (Figure 3c) ultrasonic transducer (341) replaces the electromagnetic wave receiving coil on the implant (121) inside the eye. An ultrasonic transducer (342) replaces the coil outside the eye for the ultrasonic case. A transponder (343) under the conjunctiva of the eye may be used to amplify the acoustic signal and energy either direction. By piezoelectric effects, the sound vibration is turned into electrical current, and energy extracted therefrom.

### Modulated light beam

For the light modulation (Figure 3d) case, a light emitting diode (LED) or laser diode or other light generator (361), capable of being modulated, acts as the information transmitter. Information is transferred serially by modulating the light beam, and energy is extracted from the light signal after it is converted to electricity. A photo-detector (362), such as a photodiode, which turns the modulated light signal into a modulated electrical signal, is used as a receiver. A set of a photo-generator and a photo-detector are on the implant (121) and a set is also external to the eye

### Prototype-like device

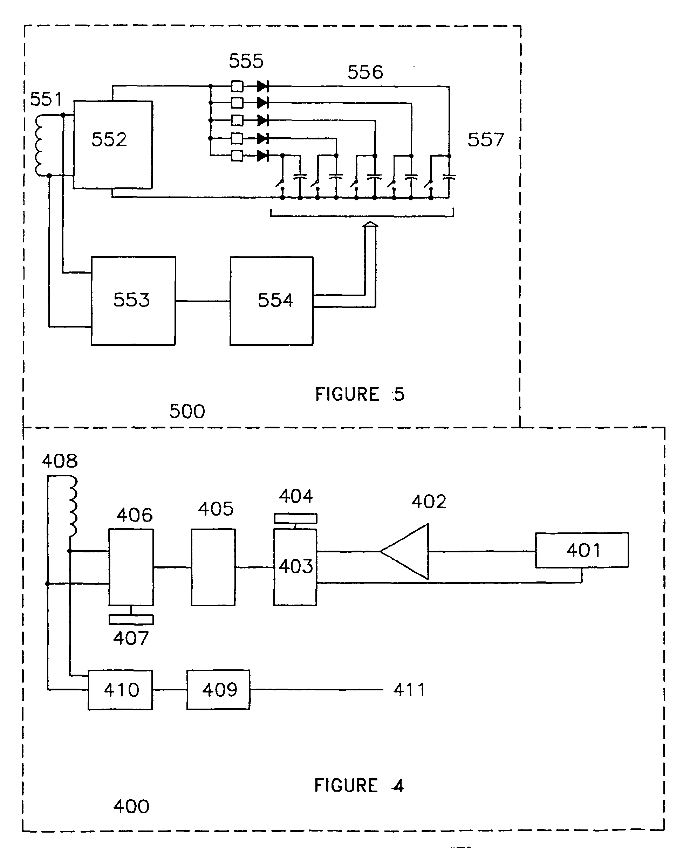

Figure 4 shows an example of the internal-to-the-eye and the external-to-the eye parts of the retinal color prosthesis, together with a means for communicating between the two. The video camera (401) connects to an amplifier (402) and to a microprocessor (403) with memory (404). The microprocessor is connected to a modulator (405). The modulator is connected to a coil drive circuit (406). The coil drive circuit is connected to an oscillator (407) and to the coil (408). The coil (408) can receive energy inductively, which can be used to recharge a battery (410), which then supplies power. The battery may also be recharged from a charger (409) on a power line source (411).

The internal-to-the eye implanted part shows a coil (551), which connects, to both a rectifier circuit (552) and to a demodulator circuit (553). The demodulator connects to a switch control unit (554). The rectifier (552) connects to a plurality of diodes (555) which rectify the current to direct current for the electrodes (556); the switch control sets the electrodes as on or off as they set the switches (557). The coils (408) and (551) serve to connect inductively the internal-to-the-eye (400) subsystem and the extemal-to-the patient (500) subsystem by electromagnetic waves. Both power and information can be sent into the internal unit. Information can be sent out to the external unit. Power is extracted from the incoming electromagnetic signal and may be accumulated by capacitors connected to each electrode or by capacitive electrodes themselves.

### Simple electrode implant

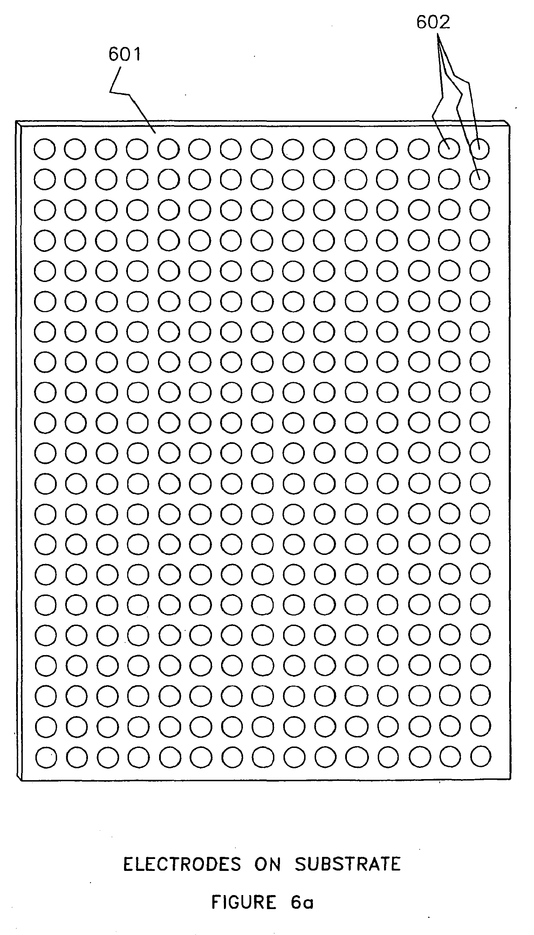

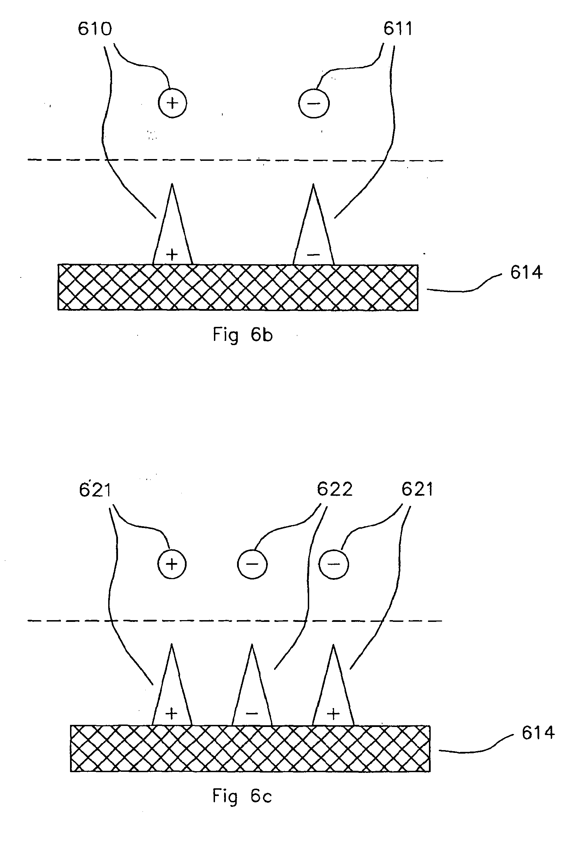

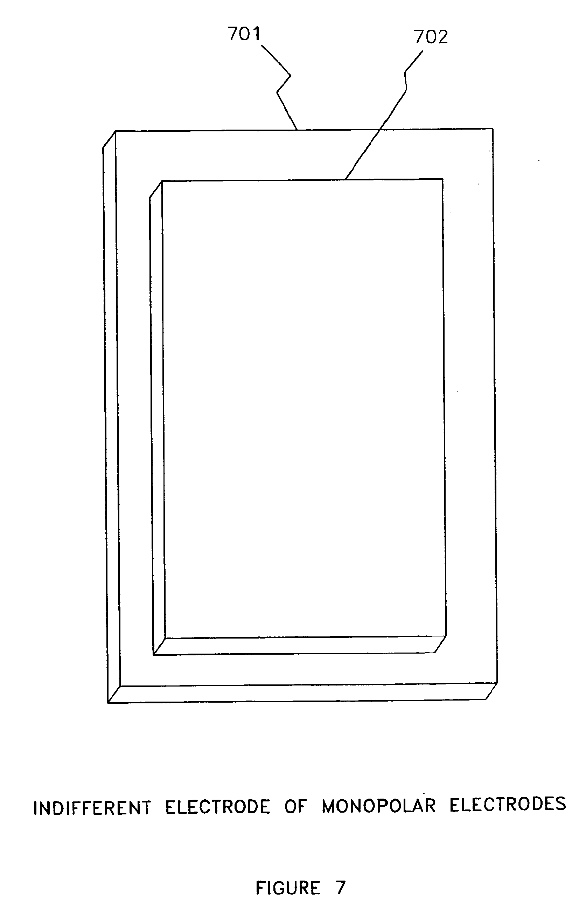

Figure 6a illustrates a set of round monopolar electrodes (602) on a substrate material (601). Figure 7 shows the corresponding indifferent electrode (702) for these monopolar electrodes, on a substrate (701), which may be the back of (601). Figure 6b shows a bipolar arrangement of electrodes, both looking down onto the plane of the electrodes, positive (610) and negative (611), and also looking at the electrodes sideways to that view, positive (610) and negative (611), sitting on their substrate (614). Similarly for Figure 6c where a multipole triplet is shown, with two positive electrodes (621) and one negative electrode, looking down on their substrate plane, and looking sideways to that view, also showing the substrate (614).

### Epiretinal electrode array

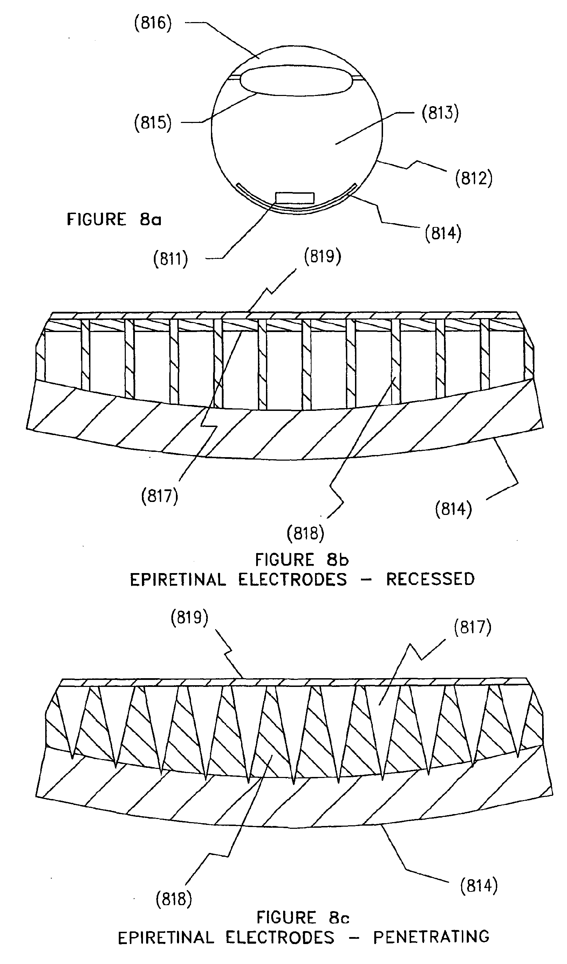

Figure 8a depicts the location of an epiretinal electrode array (811) located inside the eye (812) in the vitreous humor (813) located above the retina (814), toward the lens capsule (815) and the aqueous humor (816);
One aspect of the present embodiment, shown in Figure 8b, is the internal retinal color prosthetic part, which has electrodes (817) which may be flat conductors that are recessed in an electrical insulator (818). One flat conductor material is a biocompatible metallic foil (817). Platinum foil is a particular type of biocompatible metal foil. The electrical insulator (818) in one aspect of the embodiment is silicone.

The silicone (818) is shaped to the internal curvature of the retina (814). The vitreous humor (813), the conductive solution naturally present in the eye, becomes the effective electrode since the insulator (818) confines the field lines in a column until the current reaches the retina (814). A further advantage of this design is that the retinal tissue (814) is only in contact with the insulator (818), such as silicone, which may be more inactive, and thus, more biocompatible than the metal in the electrodes. Advantageously, another aspect of an embodiment of this invention is that adverse products produced by the electrodes (817) are distant from the retinal tissue (814) when the electrodes are recessed.

Figure 8c shows elongated epiretinal electrodes (820). The electrically conducting electrodes (820) says are contained within the electrical insulation material (818); a silicon chip (819) acts as a substrate. The electrode insulator device (818) is shaped so as to contact the retina (814) in a conformal manner.

### Subretinal electrode array

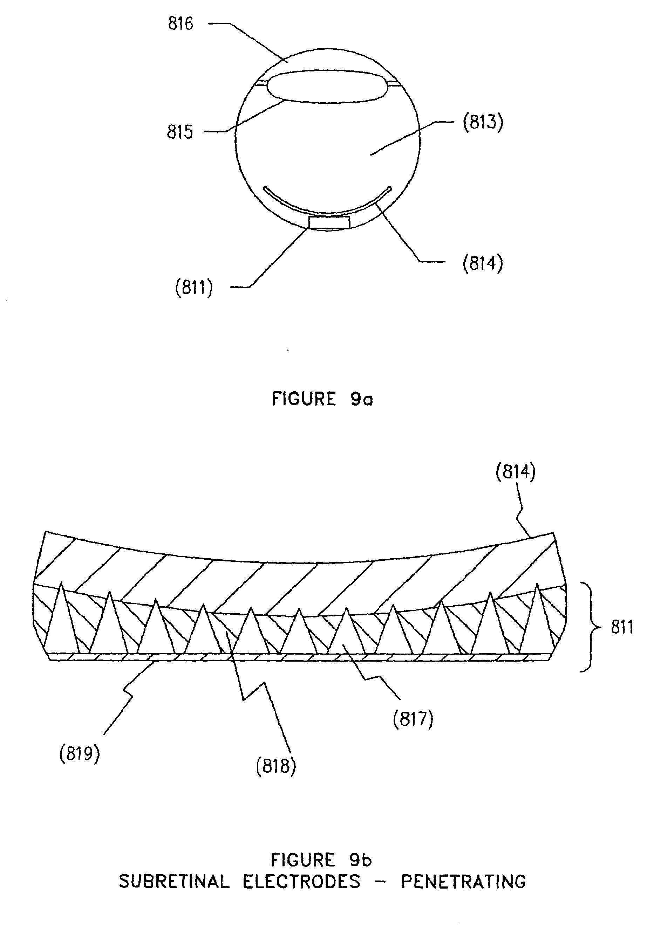

Figure 9a shows the location of a subretinal electrode array (811) below the retina (814), away from the lens capsule (815) and the aqueous humor (816). The retina (814) separates the subretinal electrode array from the vitreous humor (813). Figure 9b illustrates the subretinal electrode array (811) with pointed elongated electrodes (817), the insulator (818), and the silicon chip (819) substrate. The subretinal electrode array (811) is in conformal contact with the retina (814) with the electrodes (817) elongated to some depth.

### Electrodes

### Iridium electrodes

Now Figure 10 will illuminate structure and manufacture of iridium electrodes (Figures 10a-e). Figure 10a shows an iridium electrode, which comprises an iridium slug (1011), an insulator (1012), and a device substrate (1013). This embodiment shows the iridium slug electrode flush with the extent of the insulator. Figure 10b indicates an embodiment similar to that shown in Figure 10a, however, the iridium slug (1011) is recessed from the insulator (1012) along its sides, but with its top flush with the insulator. When the iridium electrodes (1011) are recessed in the insulating material (1012), they may have the sides exposed so as to increase the effective surface area without increasing geometric area of the face of the electrode. If an electrode (1011) is not recessed it may be coated with an insulator (1012), on all sides, except the flat surface of the face (1011) of the electrode. Such an arrangement can be embedded in an insulator that has an overall profile curvature that follows the curvature of the retina. The overall profile curvature may not be continuous, but may contain recesses, which expose the electrodes.

Figure 10c shows an embodiment with the iridium slug as in Figure 10b, however, the top of the iridium slug (1011) is recessed below the level of the insulator; Figure 10d indicates an embodiment with the iridium slug (1011) coming to a point and insulation along its sides (1021), as well as a being within the overall insulation structure (1021). Figure 10e indicates an embodiment of a method for fabricating the iridium electrodes. On a substrate (1013) of silicon an aluminum pad (1022) is deposited. On the pad the conductive adhesive (1023) is laid and platinum or iridium foil (1024) is attached thereby. A titanium ring (1025) is placed, sputtered, plated, ion implanted, ion beam assisted deposited (IBAD) or otherwise attached to the platinum or iridium foil (1024). Silicon carbide, diamond-like coating, silicon nitride and silicon oxide in combination, titanium oxide, tantalum oxide, aluminum nitride, aluminum oxide or zirconium oxide (1012) or other insulator can adhere better to the titanium (1025) while it would not otherwise adhere as well to the platinum or iridium foil (1024). The depth of the well for the iridium electrodes ranges from 0.1 µm to 1 mm.

### Elongated electrodes

Another aspect of an embodiment of the invention is the elongated electrode, which are designed to stimulate deeper retinal cells, in one embodiment, by penetrating the retina. By getting closer to the target cells for stimulation, the current required for stimulation is lower and the focus of the stimulation is more localized. The lengths chosen are 100 (m through 500 (m, including 300 (m. Figure 8c is a rendering of an elongated epiretinal electrode array with the electrodes shown as pointed electrical conductors (820), embedded in an electrical insulator (818), where the elongated electrodes (811) contact the retina in a conformal manner, however, penetrating into the retina (814).

These elongated electrodes, in an aspect of this embodiment of the invention, may be of all the same length. In a different aspect of an embodiment, they may be of different lengths. Said electrodes may be of varying lengths (Figure 8, 820), such that the overall shape of said electrode group conforms to the curvature of the retina (814). In either of these cases, each may penetrate the retina from an epiretinal position (Figure 8a, 811), or, in a different aspect of an embodiment of this invention, each may penetrate the retina from a subretinal position (Figure 9b, 817).

One method of making the elongated electrodes is by electroplating with one of an electrode material, such that the electrode, after being started, continuously grows in analogy to a stalagmite or stalactite. The elongated electrodes are 100 to 500 microns in length, the thickness of the retina averaging 200 microns. The electrode material is a substance selected from the group consisting of pyrolytic carbon, titanium nitride, platinum, iridium oxide, and iridium. The insulating material for the electrodes is a substance selected from the group silicon carbide, diamond-like coating, silicon nitride and silicon oxide in combination, titanium oxide, tantalum oxide, aluminum nitride, aluminum oxide or zirconium oxide.

### Platinum electrodes

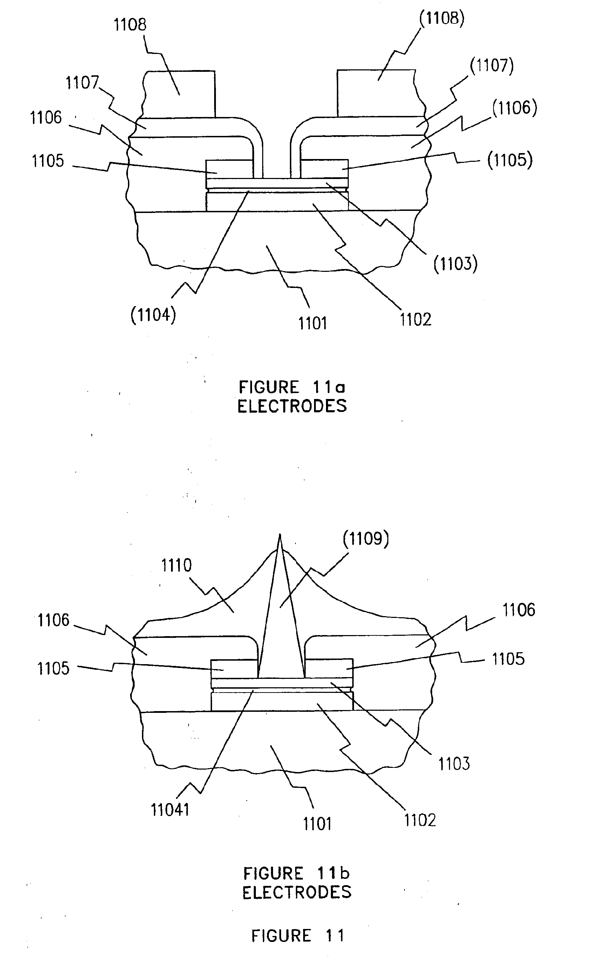

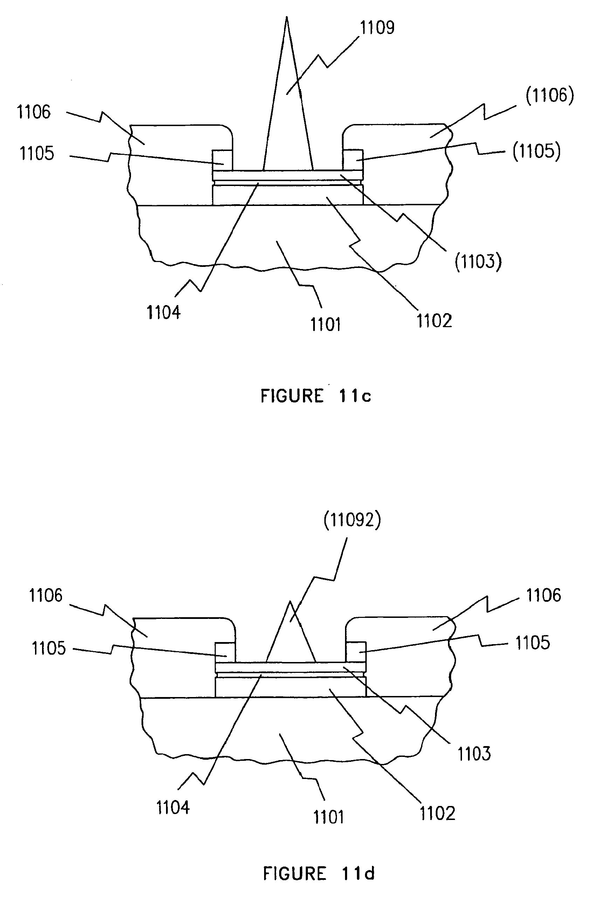

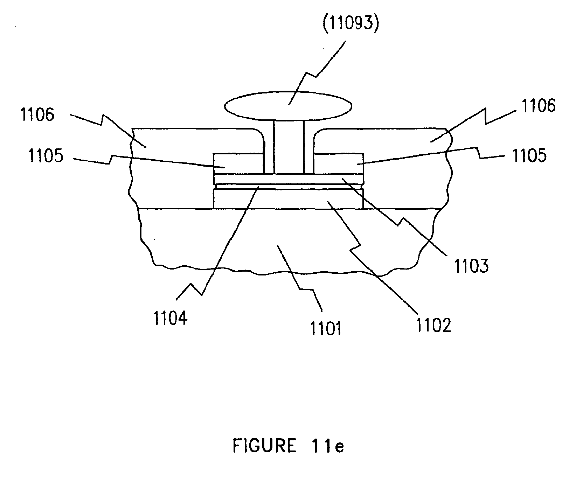

Figure 11 (a-e) demonstrates a preferred structure of, and method of, making, spiked and mushroom platinum electrodes. Examining Figure 11a one sees that the support for the flat electrode (1103) and other components such as electronic circuits (not shown) is the silicon substrate (1101). An aluminum pad (1102) is placed where an electrode or other component is to be placed (1102). In order to hermetically seal-off the aluminum and silicon from any contact with biological activity, a metal foil (1103), such as platinum or iridium, is applied to the aluminum pad (1102) using conductive adhesive (1104). Electroplating is not used since a layer formed by electroplating, in the range of the required thinness, has small-scale defects or holes which destroy the hermetic character of the layer. A titanium ring (1105) is next placed on the platinum or iridium foil (1103). Normally, this placement is by ion implantation, sputtering or ion beam assisted deposition (IBAD) methods. Silicon carbide, diamond-like coating, silicon nitride and silicon oxide in combination, titanium oxide, tantalum oxide, aluminum nitride, aluminum oxide or zirconium oxide (1106) is placed on the silicon substrate (1101) and the titanium ring (1105). In one embodiment, an aluminum layer (1107) is plated onto exposed parts of the titanium ring (1105) and onto the silicon carbide, diamond-like coating, silicon nitride and silicon oxide in combination, titanium oxide, tantalum oxide, aluminum nitride, aluminum oxide or zirconium oxide (1106). In this embodiment the aluminum (1107) layer acts as an electrical conductor. A mask (1108) is placed over the aluminum layer (1107).

In forming an elongated, non-flat, electrode platinum (Figure 11b) is electroplated onto the platinum or iridium foil (1103). Subsequently, the mask (1108) is removed and insulation (1110) is applied over the platinum electrode (1109).

In Figure 11c a platinum electrode (1109) is shown which is more internal to the well formed by the silicon carbide, diamond-like coating, silicon nitride and silicon oxide in combination, titanium oxide, tantalum oxide, aluminum nitride, aluminum oxide or zirconium oxide and its titanium ring. The electrode (1109) is also thinner and more elongated and more pointed. Figure 11d shows a platinum electrode formed by the same method as was used in Figures 11a, 11b, and 11c. The platinum electrode (1192) is more internal to the well formed by the silicon carbide, diamond-like coating, silicon nitride and silicon oxide in combination, titanium oxide, tantalum oxide, aluminum nitride, aluminum oxide or zirconium oxide and its titanium ring as was the electrode (1109) in Figure 11c. However it is less elongated and less pointed.

The platinum electrode is internal to the well formed by the silicon carbide, diamond-like coating, silicon nitride and silicon oxide in combination, titanium oxide, tantalum oxide, aluminum nitride, aluminum oxide or zirconium oxide and its titanium ring; said electrode whole angle at it's peak being in the range from 1° to 120°; the base of said conical or pyramidal electrode ranging from 1 µm to 500 µm; the linear section of the well unoccupied by said conical or pyramidal electrode ranging from zero to one-third.

A similar overall construction is depicted in Figure 11 e. The electrode (1193), which may be platinum, is termed a mushroom shape. The maximum current density for a given metal is fixed. The mushroom shape presents a relatively larger area than a conical electrode of the same height. The mushroom shape advantageously allows a higher current, for the given limitation on the current density (e.g., milliamperes per square millimeter) for the chosen electrode material, since the mushroom shape provides a larger area.

### Inductive coupling coils

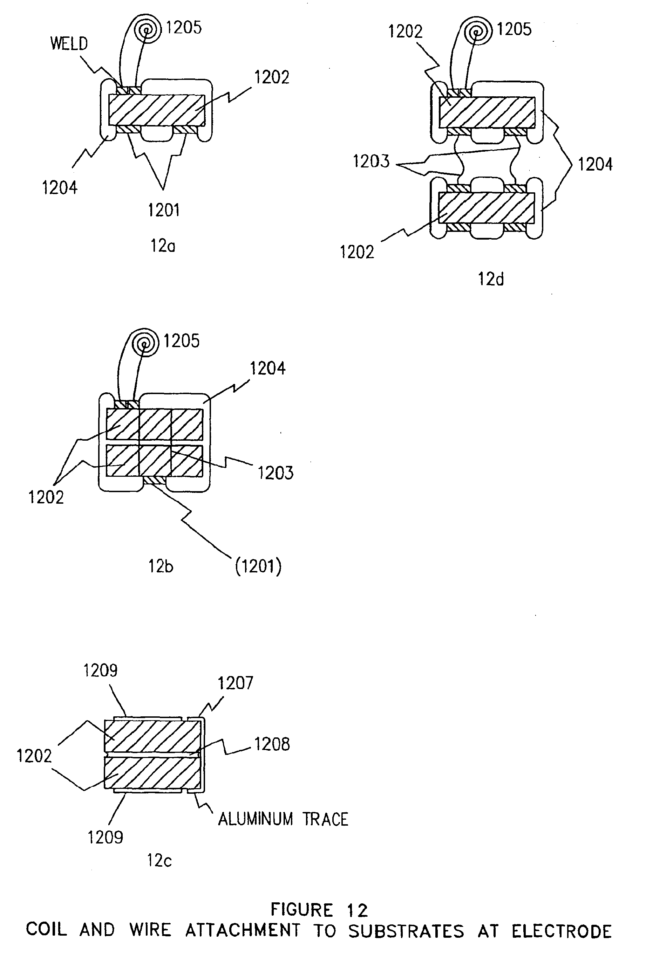

Information transmitted electromagnetically into or out of the implanted retinal color prosthesis utilizes insulated conducting coils so as to allow for inductive energy and signal coupling. Figure 12 shows an insulated conducting coil and insulated conducting electrical pathways, e.g., wires, attached to substrates at what would otherwise be electrode nodes, with flat, recessed metallic, conductive electrodes (1201). In referring to wire or wires, insulated conducting electrical pathways are included, such as in a "two-dimensional" "on-chip" coil or a "two-dimensional" coil on a polymide substrate, and the leads to and from these "two-dimensional" coil structures. A silicon carbide, diamond-like coating, silicon nitride and silicon oxide in combination, titanium oxide, tantalum oxide, aluminum nitride, aluminum oxide or zirconium oxide (1204) is shown acting as both an insulator and an hermetic seal. Another aspect of the embodiment is shown in Figure 12a. The electrode array unit (1201) and the electronic circuitry unit (1202) can be on one substrate, or they may be on separate substrates (1202) joined by an insulated wire or by a plurality of insulated wires (1203). Said separate substrate units can be relatively near one another. For example they might lie against a retinal surface, either epiretinally or subretinally placed. Two substrates units connected by insulated wires may carry more electrodes than if only one substrate with electrodes was employed, or it might be arranged with one substrate carrying the electrodes, the other the electronic circuitry. Another arrangement has the electrode substrate or substrates placed in a position to stimulate the retinal cells, while the electronics are located closer to the lens of the eye to avoid heating the sensitive retinal tissue.

In all of the Figures 12a, 12b, and 12c, a coil (1205) is shown attached by an insulated wire. The coil can be a coil of wire, or it can be a "two dimensional" trace as an "on-chip" component or as a component on polyimide. This coil can provide a stronger electromagnetic coupling to an outside-the-eye source of power and of signals. Figure 12d shows an externally placed aluminum (conductive) trace instead of the electrically conducting wire of Figure 12c. Also shown is an electrically insulating adhesive (1208) which prevents electrical contact between the substrates (1202) carrying active circuitry (1209).

### Hermetic sealing

### Hermetic coating

All structures, which are subject to corrosive action as a result of being implanted in the eye, or, those structures which are not completely biocompatible and not completely safe to the internal cells and fluids of the eye require hermetic sealing. Hermetic sealing may be accomplished by coating the object to be sealed with silicon carbide, diamond-like coating, silicon nitride and silicon oxide in combination, titanium oxide, tantalum oxide, aluminum nitride, aluminum oxide or zirconium oxide. These materials also provide electrical insulation. The method and apparatus of hermetic sealing by aluminum and zirconium oxide coating is described in a pending U. S. Patent Application, Serial Number 08/994515. The methods of coating a substrate material with the hermetic sealant include sputtering, ion implantation, and ion-beam-assisted deposition (IBAD).

### Hermetic box

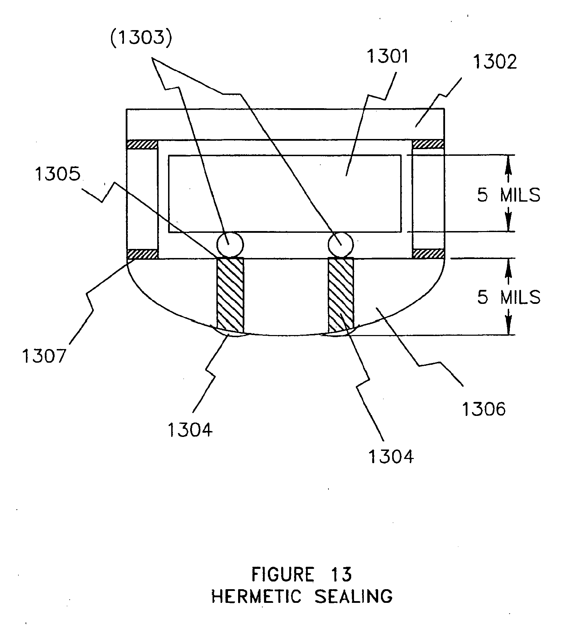

Another aspect of an embodiment of the invention is hermetically sealing the silicon chip (1301) by placing it in a metal or ceramic box (1302) of rectangular cross-section with the top and bottom sides initially open (Figure 13). The box may be of one (1302) of the metals selected from the group comprising platinum, iridium, palladium, gold, and stainless steel. Solder balls (1303) are placed on the "flip-chip", i.e., a silicon-based chip that has the contacts on the bottom of the chip (1301). Metal feedthroughs (1304) made from a metal selected from the group consisting of radium, platinum, titanium, iridium, palladium, gold, and stainless steel. The bottom cover (1306) is formed from one of the ceramics selected from the group consisting of aluminum oxide or zirconium oxide. The inner surface (1805), toward the solder ball, (1803)) of the feed-through (1804) is plated with gold or with nickel. The ceramic cover (1806) is then attached to the box using a braze (1807) selected from the group consisting of: 50% titanium together with 50% nickel and gold. Electronics are then inserted and the metal top cover (of the same metal selected for the box) is laser welded in place.

### Separate electronics chip substrate and electrode substrate

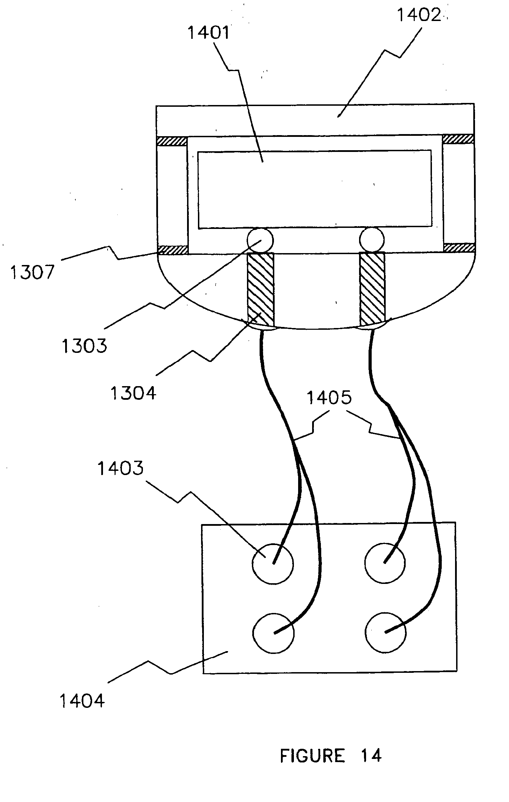

In one embodiment of the invention (Figure 14), the chip substrate (1401) is hermetically sealed in a case (1402) or by a coating of the aluminum, zirconium, or magnesium oxide coating. However, the electrodes (1403) and its substrate (1404) form a distinct and separate element. Insulated and hermetically sealed wires (1405) connect the two. The placement of the electrode element may be epiretinal, while the electronic chip element may be relatively distant from the electrode element, as much distant as being in the vicinity of the eye lens. Another embodiment of the invention has the electrode element placed subretinally and the electronic chip element placed toward the rear of the eye, being outside the eye, or, being embedded in the sclera of the eye or in or under the choroid, blood support region for the retina. Another embodiment of the invention has the electronic chip element implanted in the fatty tissue behind the eye and the electrode element placed subretinally or epiretinally.

### Capacitive electrodes

A plurality of capacitive electrodes can be used to stimulate the retina, in place of non-capacitive electrodes. A method of fabricating said capacitive electrode uses a pair of substances selected from the pair group consisting of the pairs iridium and iridium oxide; and, titanium and titanium nitride. The metal electrode acts with the insulating oxide or nitride, which typically forms of its own accord on the surface of the electrode. Together, the conductor and the insulator form an electrode with capacitance.

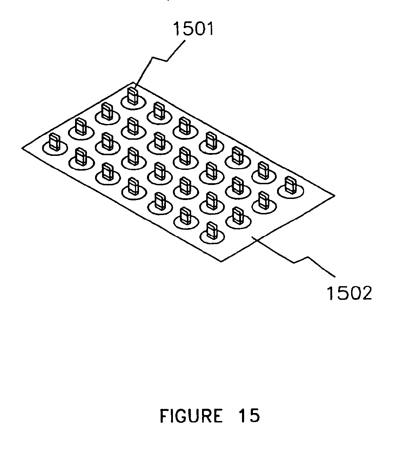

Mini-capacitors (Figure 15) can also be used to supply the required isolating capacity. The capacity of the small volume size capacitors (1501) is 0.47 microfarads. The dimensions of these capacitors are individually 20 mils (length) by 20 mils (width) by 40 mils (height). In one embodiment of the invention, the capacitors are mounted on the surface of a chip substrate (1502), that surface being opposite to the surface containing the active electronics elements of the chip substrate.

### Electrode/electronics component placement

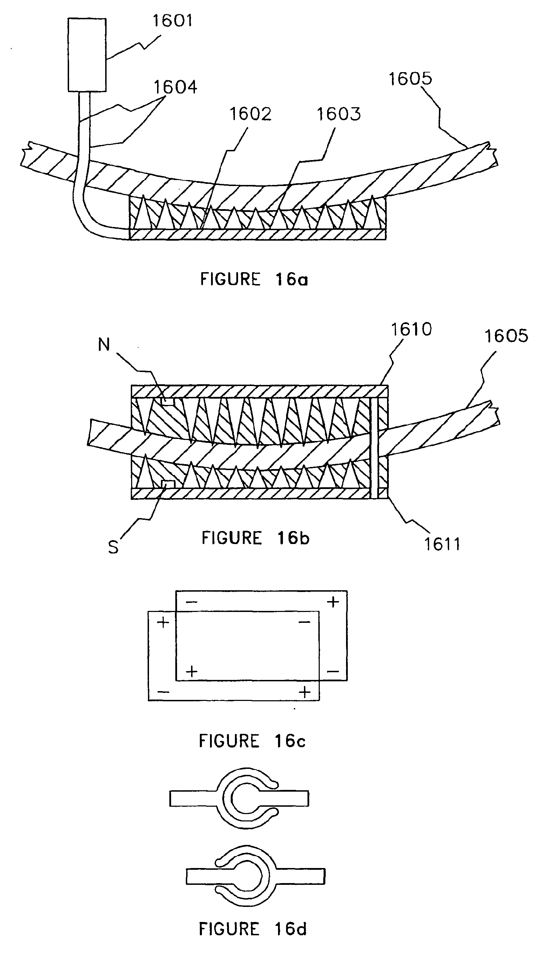

In one embodiment (Figure 16a) the internal-to-the-eye-implanted part consists of two subsystems, the electrode component subretinally positioned and the electronic component epiretinally positioned. The electronics component, with its relatively high heat dissipation, is positioned at a distance, within the eye, from the electrode component placed near the retina that is sensitive to heat.

An alternative embodiment shown in Figure 16b is where one of the combined electronic and electrode substrate units is positioned subretinally and the other is located epiretinally and both are held together across the retina so as to efficiently stimulate bipolar and associated cells in the retina.

An alternative embodiment of the invention has the electronic chip element implanted in the fatty tissue behind the eye and the electrode element placed subretinally or epiretinally, and power and signal communication between them by electromagnetic means including radio-frequency (rf), optical, and quasi-static magnetic fields, or by acoustic means including ultrasonic transducers.

Figure 16c shows how the two electronic-electrode substrate units are held positioned in a prescribed relationship to each other by small magnets. Alternatively the two electronic-electrode substrate units are held in position by alignment pins. Another aspect of this is to have the two electronic-electrode substrate units held positioned in a prescribed relationship to each other by snap-together mating parts, some exemplary ones being shown in Figure 16d.

### Neurotrophic factor

Another aspect of the embodiment is the use of a neurotrophic factor, for example, Nerve Growth Factor, applied to the electrodes, or to the vicinity of the electrodes, to aid in attracting target nerves and other nerves to grow toward the electrodes.

### Eye-motion compensation system

Another aspect of the embodiment is an eye-motion compensation system comprising an eye-movement tracking apparatus (Figure 1b, 112); measurements of eye movement; a transmitter to convey said measurements to video data processor unit that interprets eye movement measurements as angular positions, angular velocities, and angular accelerations; and the processing of eye position, velocity, acceleration data by the video data processing unit for image compensation, stabilization and adjustment.

Ways of eye-tracking (Figure 1b, 112) include utilizing the corneal eye reflex, utilizing an apparatus for measurements of electrical activity where one or more coils are located on the eye and one or more coils are outside the eye, utilizing an apparatus where three orthogonal coils placed on the eye and three orthogonal coils placed outside the eye, utilizing an apparatus for tracking movements where electrical recordings from extra-ocular muscles are measured and conveyed to the video data processing unit that interprets such electrical measurements as angular positions, angular velocities, and angular accelerations. The video data processing unit uses these values for eye position, velocity, and acceleration to compute image compensation, stabilization and adjustment data, which is then applied by the video data processor to the electronic form of the image.

### Head sensor

Another aspect of the embodiment utilizes a head motion sensor (131). The basic sensor in the head motion sensor unit is an integrating accelerometer. A laser gyroscope can also be used. A third sensor is the combination of an integrating accelerometer and a laser gyroscope. The video data processing unit can incorporate the data of the motion of the eye as well as that of the head to further adjust the image electronically so as to account for eye motion and head motion.

### Physician's local control unit

Another aspect includes a retinal prosthesis with (see Figure 1a) a physician's local external control unit (15) allowing the physician to exert setup control of parameters such as amplitudes, pulse widths, frequencies, and patterns of electrical stimulation. The physician's control unit (15) is also capable of monitoring information from the implanted unit (21) such as electrode current, electrode impedance, compliance voltage, and electrical recordings from the retina. The monitoring is done via the internal telemetry unit, electrode and electronics assembly (21).

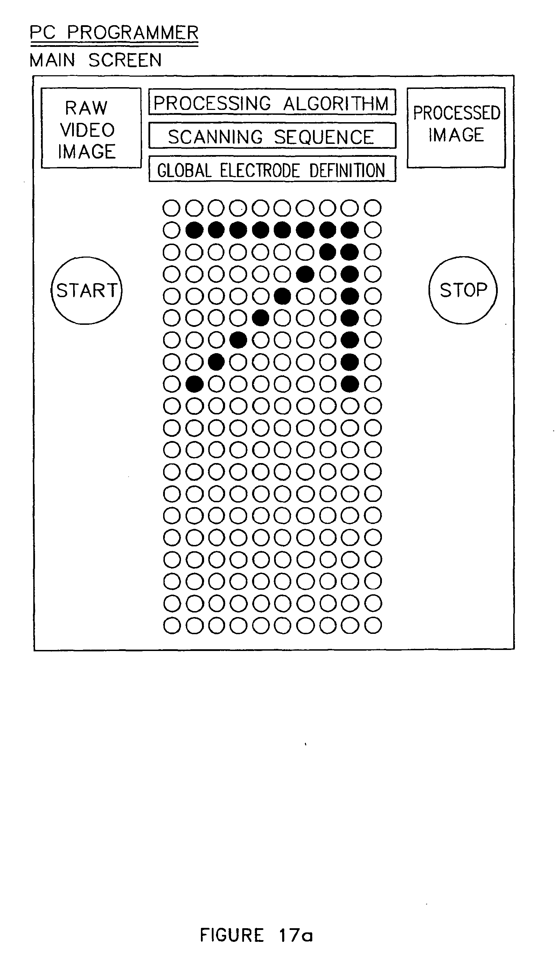

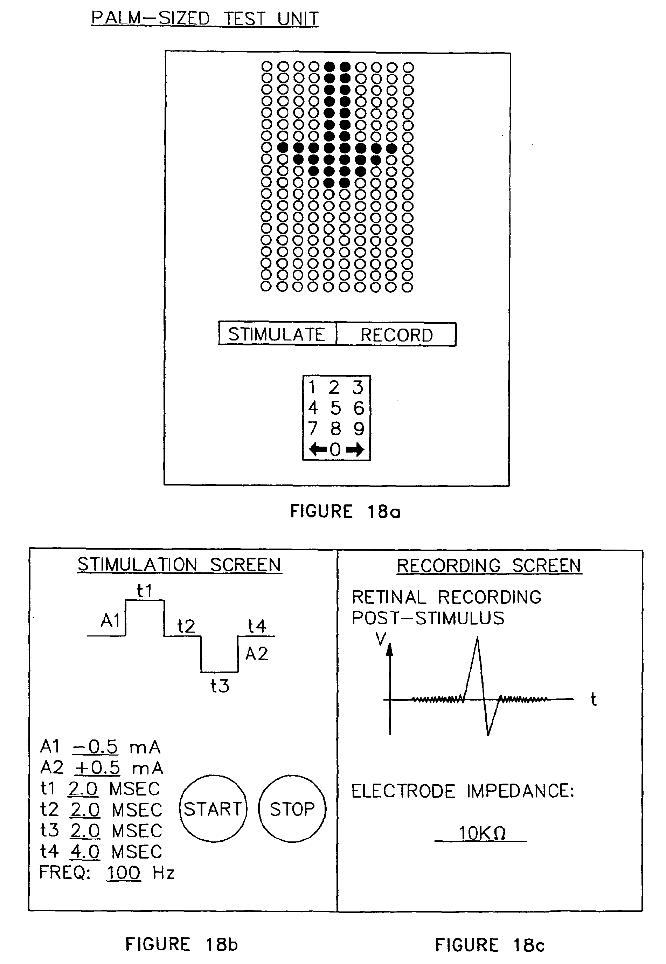

An important aspect of setting up the retinal color prosthesis is setting up electrode current amplitudes, pulse widths, and frequencies so they are comfortable for the patient. Figures 17a-c and Figures 18a-c illustrate some of the typical displays. A computer-controlled stimulating test that incorporates patient response to arrive at optimal patient settings may be compared to being fitted for eyeglasses, first determining diopter, then cylindrical astigmatic correction, and so forth for each patient. The computer uses interpolation and extrapolation routines. Curve or surface or volume fitting of data may be used. For each pixel, the intensity in increased until a threshold is reached and the patient can detect something in his visual field. The intensity is further increased until the maximum comfortable brightness is reached. The patient determines his subjective impression of one-quarter maximum brightness, one-half maximum brightness, and three-quarters maximum brightness. Using the semi-automated processing of the patient-in-the-loop with the computer, the test program runs through the sequences and permutations of parameters and remembers the patient responses. In this way apparent brightness response curves are calibrated for each electrode for amplitude. Additionally, in the same way as for amplitude, pulse width and pulse rate (frequency), response curves are calibrated for each patient. The clinician can then determine what the best settings are for the patient.

This method is generally applicable to many, if not all, types of electrode based retinal prostheses. Moreover, it also is applicable to the type of retinal prosthesis, which uses an external light intensifier shining upon essentially a spatially distributed set of light sensitive diodes with a light activated electrode. In this latter case, a physician's test, setup and control unit is applied to the light intensifier which scans the implanted photodiode array, element by element, where the patient can give feedback and so adjust the light intensifier parameters.

### Remote physician's unit

Another aspect of an embodiment of this invention includes (see Figure 1b) a remote physician control unit (117) that can communicate with a patient's unit (114) over the public switched telephone network or other telephony means. This telephone-based pair of units is capable of performing all of the functions of the of the physician's local control unit (115).

### Physician's unit measurements, menus and displays

Both the physician's local (115) and the physician's remote (117) units always measure brightness, amplitudes, pulse widths, frequencies, patterns of stimulation, shape of log amplification curve, electrode current, electrode impedance, compliance voltage and electrical recordings from the retina.

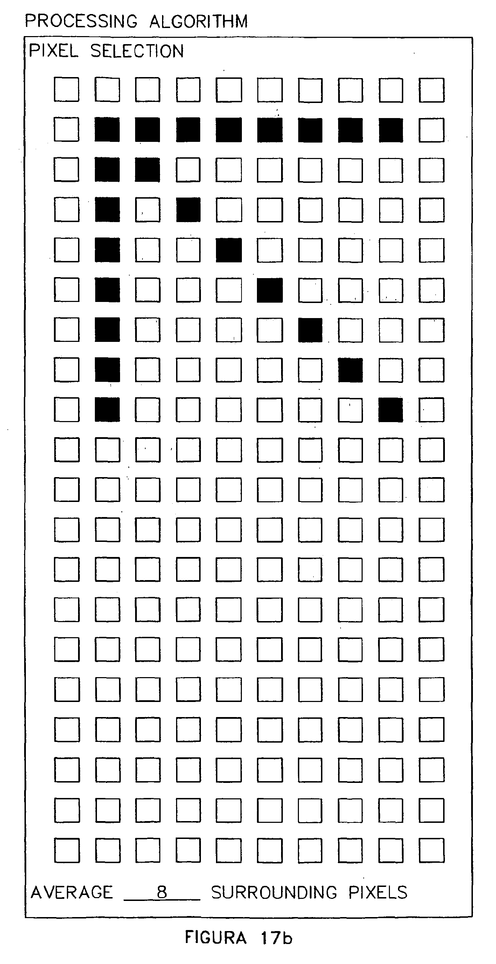

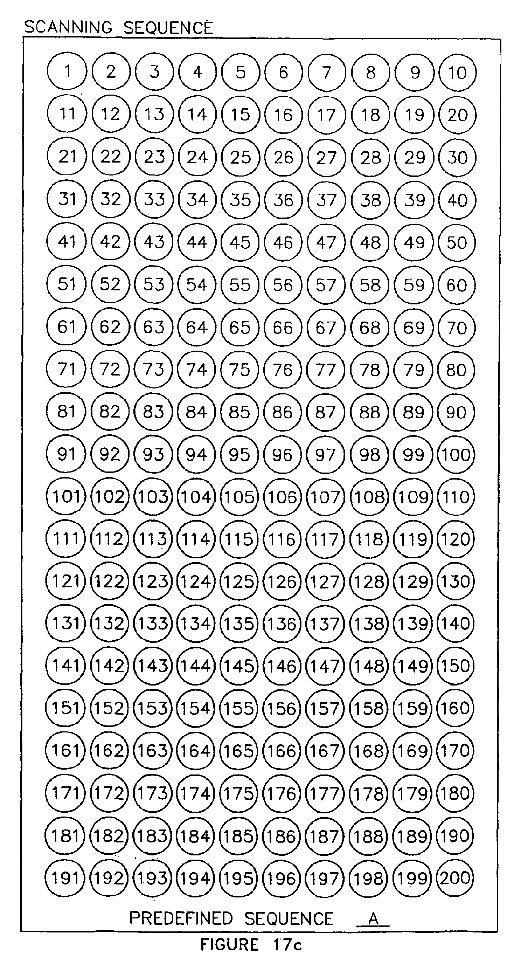

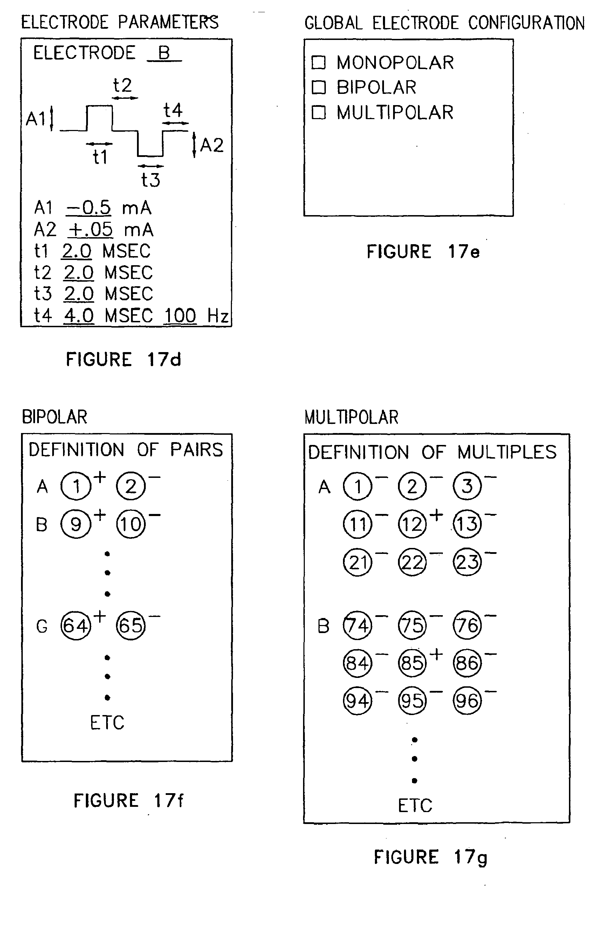

Figure 17a shows the main screen of the Physician's Local and Remote Controller and Programmer. Figure 17b illustrates the pixel selection of the processing algorithm with the averaging of eight surrounding pixels chosen as one element of the processing. Figure 17c represents an electrode scanning sequence, in this case the predefined sequence, A. Figure 17d shows electrode parameters, here for electrode B, including current amplitudes and waveform timelines. Figure 17e illustrates the screen for choosing the global electrode configuration, monopolar, bipolar, or multipolar. Figure 17f renders a screen showing the definition of bipolar pairs (of electrodes). Figure 17g shows the definition of the multipole arrangements.

Figure 18a illustrates the main menu screen for the palm-sized test unit. Figure 18b shows a result of pressing on the stimulate bar of the (palm-sized unit) main menu screen, where upon pressing the start button the amplitudes A1 and A2 are stimulated for times t1, t2, t3, and t4, until the stop button is pressed. Figure 18c exhibits a recording screen that shows the retinal recording of the post-stimulus and the electrode impedance.

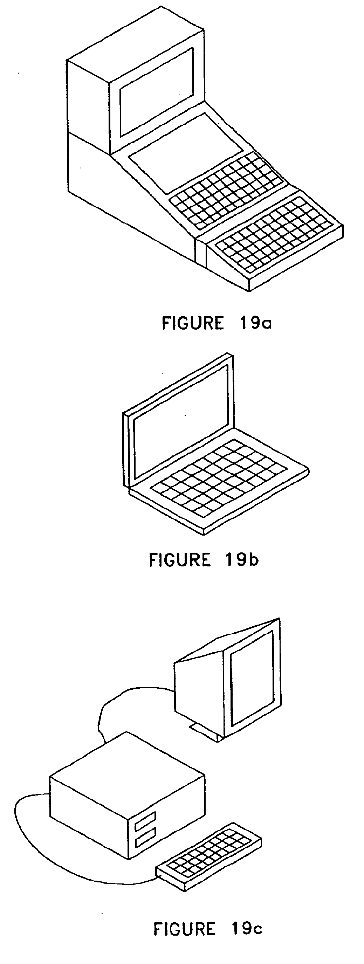

Figure 19a, 19b and 19c show different embodiments of the Physician's Remote Controller, which has the same functionality inside as the Physician's Local Controller but with the addition of communication means such as telemetry or telephone modem.

### Patient's controller

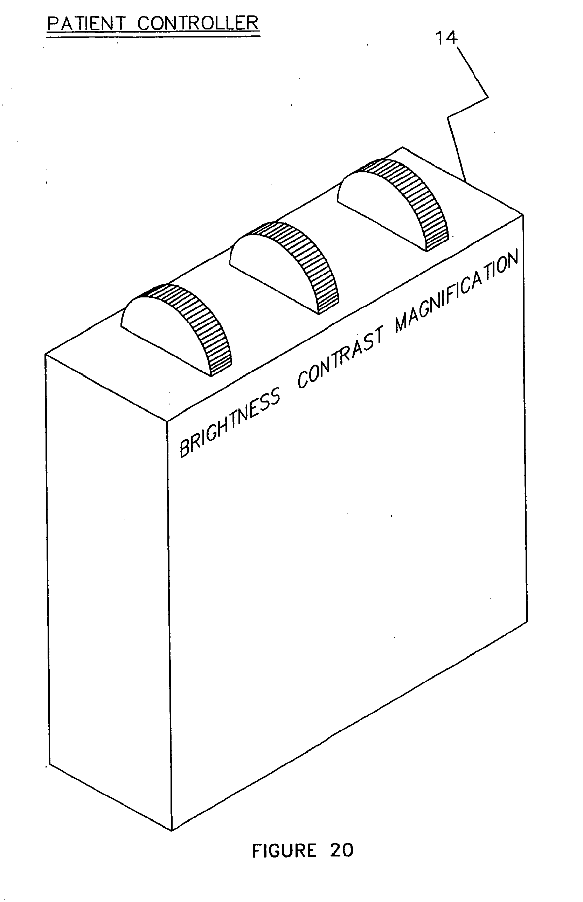

Corresponding to the Physician's Local Controller, but with much less capability, is the Patient's Controller. Figure 20 shows the patient's local controller unit. This unit can monitor and adjust brightness (2001), contrast (2002) and magnification (2003) of the image on a non-continuous basis. The magnification control (2003) adjusts magnification both by optical zoom lens control of the lens for the imaging means (Figure 1, 11), and by electronic adjustment of the image in the data processor (Figure 2, 13).

While the invention herein disclosed has been described by means of specific embodiments and applications thereof, numerous modifications and variations could be made thereto by those skilled in the art without departing from the scope of the invention set forth in the claims.

The following numbered paragraphs contain statements of broad combinations of technical features in accordance with various aspects of the devices, apparatus and methods disclosed herein:
1. A retinal prosthetic for color sight restoration comprising:
   a. a color imager to capture color image, said color imaging being part of an external component, which is carried by the patient;
   b. a video data processing unit for converting said image to electrical signals which contain image color signal data, said color imaging being part of said external component, which is carried by the patient;
   c. a plurality of electrodes and one or more electronic circuits, implantable in the eye, said electrode and said electronic circuits being part of a component which is internal to the eye;
   d. an external and internal receiver and transmitter for communication between the external component and the internal components;
   e. said implantable electronic circuits configured to receive color signal information from the video data processing unit, activate the electrodes based on said color signal information and extract electrical power from the received signal;
   f. said electrodes configured to stimulate those cells of the retina electrically which when stimulated give the perception of color.
2. The retinal color prosthesis as in paragraph 1 further comprising a video data processing unit which converts said color image to electrical signals corresponding to data for a pixel or grid-like arrangement of electrodes placed within the eye.
3. The retinal color prosthesis as in paragraph 2 further comprising said video data processing unit wherein data processing algorithms and processing hardware include data processing for electronic compensation for the motion of the eye (s) and head are processed.
4. The retinal color prosthesis as in paragraph 3 further comprising an eye-motion compensation system.
5. The retinal color prosthesis as in paragraph 4 further comprising an eye-movement tracking apparatus; data output of said eye-movement tracking apparatus; wherein said data corresponds to measurements of eye movement; a transmitter to convey said measurement data to said video data processing unit; wherein the video data processing unit algorithmically processes eye movement measurements such as angular positions, angular velocities, and angular accelerations; temporarily stored data image compensation parameters, stabilization parameters, and adjustment parameters from the processed eye position, eye velocity, and eye acceleration, electronic image stabilization to provide eye-motion compensation computationally derived from said temporarily stored data whereby the electronic image presented to the implanted electrodes inside the eye corresponds to what a normal eye would see when looking in its direction.
6. The retinal color prosthesis as in paragraph 5 further comprising a head tracking system wherein a basic sensor in the head motion sensor unit is a sensor selected from the group consisting of an integrating accelerometer, a micro-machined mechanical gyroscope, a laser gyroscope, a combination of an integrating accelerometer and a micro-machined mechanical gyroscope; a combination of an integrating accelerometer and a laser gyroscope; the motion and position of the head is determined by said basic sensor; wherein the data are communicated from the head tracking system to the video data processing unit by telemetry; wherein the data are processed in the video data processor; and wherein said video data processing unit can process the data of the motion of the eye as well as that of the head to further adjust the image electronically whereby the electronic data image is presented to the patient adjusted for eye motion and head motion.
7. The retinal color prosthesis as in paragraph 1 further comprising programmable external-to-the-patient and internal-to-the-eye units which monitor and adjust, upon external command, data to and from the internal-to-the eye retinal implants.
8. The retinal color prosthesis as in paragraph 7 further comprising a physician's control unit located external to the patient which can controls image data signal information sent from the video data processing unit into the internal-to-the-eye implant; and wherein the physician's control unit can receive information which the internal-to-the-eye implant transmits out of the eye.
9. The retinal color prosthesis as in paragraph 8 further comprising said unit wherein the physician can control parameters of the image data signal such as amplitudes, pulse widths, frequencies, and patterns of electrical stimulation.
10. The retinal color prosthesis as in paragraph 9 wherein said physician's control unit monitors said transmitted-out information including electrode current, electrode impedance, compliance voltage, or, electrical recordings from the retina.
11. The retinal color prosthesis as in paragraph 7 further comprising a physician's hand-held test unit comprising a hand-held computer used to set up and evaluate a retinal prosthesis implant during or soon after implantation at the patient's bedside; said hand-held computer having the capability of receiving what signals are transmitted out of the eye and having the ability to send information in to the retinal implant electronic chip; said hand-held computer able to adjust the amplitudes on each electrode, one at a time, or in groups.
12. The retinal color prosthesis as in paragraph 7 further comprising a patient's control unit wherein a unit located external to the eye controls apparent brightness, contrast and magnification as presented to the patient by the retinal prosthesis; that has circuitry, which upon remote command, will respond in a manner similar to that of said physician's local unit; and which has a transmitter and a receiver for remote communication.
13. The retinal color prosthesis as in paragraph 11 further comprising the physician's remote unit further comprising a transmitter and a receiver for communication with the video data processing unit wherein said physician's remote unit performs all of the functions of the local physician's control unit.
14. The retinal color prosthesis as in paragraph 2 further comprising said video data processing unit for encoding and transmitting time sequences, amplitudes and widths of pulses for signaling color to retinal cells.
15. The retinal color prosthesis as in paragraph 2 further comprising said video data processing unit for encoding electrical signal information for signaling color to the different retinal cells, in a planar pattern, corresponding to the location of electrodes in the vicinity of the individual retinal cells, including red-center-green surround, green-center-red-surround, blue-center-yellow surround and yellow-centerblue-surround bipolar cells.
16. The retinal color prosthesis as in paragraph 1 further comprising one or more hermetically sealed and partially insulated electrode subpart (s).
17. The retinal color prosthesis as in paragraph 1 further comprising one or more hermetically sealed electronic circuit subpart (s).
18. The retinal color prosthesis as in paragraph 1 further comprising electronic circuits within the eye fortransferring received processed image data to the electrodes.
19. The retinal color prosthesis as in paragraph 18 further comprising electronic circuits for applying received color signal information to a plurality of electrodes, corresponding to the original external pixel-like scene capture.
20. The retinal color prosthesis as in paragraph 1 further comprising electronic circuits which measure and transmit out of the eye the signal power level being received internally.
21. The retinal color prosthesis as in paragraph 1 further comprising electronic circuits which measure and transmit out of the eye an impedance for each of the different electrodes.
22. The retinal color prosthesis as in paragraph 1 further comprising electronic circuits which measure electrophysiologic retinal recordings and transmit them out of the eye.
23. The retinal color prosthesis as in paragraph 1 further comprising said internal component at least partially implanted subretinally.
24. The retinal color prosthesis as in paragraph 1 further comprising said internal component implanted epiretinally.
25. The retinal color prosthesis as in paragraph 1 further comprising said plurality of electrodes in a monopolar arrangement.
26. The retinal color prosthesis as in paragraph 1 further comprising said plurality of electrodes in a bipolar arrangement.
27. The retinal color prosthesis as in paragraph 1 further comprising said plurality of electrodes in a multipolar arrangement.
28. The retinal color prosthesis as in paragraph 27 further comprising said plurality of electrodes in an electric field focusing arrangement.
29. The retinal color prosthesis as in paragraph 1 wherein said plurality of electrodes further comprises a substance from the group consisting of pyrolytic carbon, titanium nitride, platinum, iridium, and iridium oxide.
30. The retinal color prosthesis as in paragraph 16 or paragraph 17 further comprising an hermetic sealant coating substance selected from the group consisting of silicon carbide, diamond-like coating, silicon nitride and silicon oxide in combination, titanium oxide, tantalum oxide, aluminum nitride, aluminum oxide or zirconium oxide.
31. The retinal color prosthesis as in paragraph 1 further comprising said plurality of electrodes insulated along their extension, up to their tips.
32. The retinal color prosthesis as in paragraph 31 further comprising said insulation selected from the group consisting of silicon carbide, diamond-like coating, silicon nitride and silicon oxide in combination, titanium oxide, tantalum oxide, aluminum nitride, aluminum oxide, zirconium oxide, PTFE (polytetrafluoroethylene), FEP (fluorinated ethylene propylene and waxes.
33. The retinal color prosthesis as in paragraph 1 comprising a plurality of capacitive electrodes, formed from a pair of substances, selected from the group of pairs consisting of the pairs iridium and iridium oxide, and, titanium and titanium nitride.
34. The retinal color prosthesis as in paragraph 1 comprising a plurality of capacitors which are mounted on the electrode substrate in a one-to-one correspondence with the plurality of electrodes.
35. The retinal color prosthesis as in paragraph 1 further comprising said internal component having a configuration of two separate parts wherein insulated wires join said parts.
36. The retinal color prosthesis as in paragraph 35 further comprising one of said two separate parts of the internal component that is configured as a combined electronic and electrode component and the other of said two separate parts of the internal component which is also configured as a combined electronic and electrode component.
37. The retinal color prosthesis as in paragraph 36 further comprising one of said combined electronic and electrode component subretinally positioned and the other of said combined electronic and electrode component epiretinally positioned.
38. The retinal color prosthesis as in paragraph 37 wherein said two components are held positioned in a prescribed relationship to each other by small magnets.
39. The retinal color prosthesis as in paragraph 37 wherein said two components are held positioned in a prescribed relationship to each other by alignment pins.
40. The retinal color prosthesis as in paragraph 37 wherein said two components are held positioned in a prescribed relationship to each other by snap together mating parts.
41. The retinal color prosthesis as in paragraph 35 further comprising one of said two separate parts of the internal component that is configured as an electronic component and the other of said two separate parts of the internal component which is configured as an electrode component.
42. The retinal color prosthesis as in paragraph 41 further comprising said electrode component subretinally positioned and said electronic component epiretinally positioned.
43. The retinal color prosthesis as in paragraph 42 further comprising said electronics component in a position distant, within the eye, from said electrode component placed near the retina.
44. The retinal color prosthesis as in paragraph 1 further comprising an insulated conducting coil attached to the internal component, each end of the coil attached to an electrically different attachment point on said internal component.
45. The retinal color prosthesis as in paragraph 44 further comprising two insulated coils, one larger, and the second smaller, one free end of one coil joined to one free end of the second coil, the other free end of said one coil joined to the other free end of said second coil, said smaller coil in proximity to a coil attached to said internal component, said larger coil positioned and fastened toward the lens of the eye, for example, by suturing.
46. The retinal color prosthesis as in paragraph 1 further comprising internal component which is split into two parts, first part an electronics part, the second part an electronics and electrode part; the first and second parts have integral to each a pick-up of conducting insulated conducting coil; an energy transfer structure comprising two insulated conducting coils, one larger, and the second smaller, one free end of one coil joined to one free end of the second coil, the other free end of said one coil joined to the other free end of said second coil, said smaller coil in proximity to said coil attached to the first internal part; said first internal part located in the fatty tissue behind the eye; the second internal part located within the eye; said larger coil attached temporally in the vicinity of the eye.
47. The retinal color prosthesis as in paragraph 1 further comprising a coating of neurotrophic factor on the surface of the electrodes.
48. The retinal color prosthesis as in paragraph 47 further comprising a coating of Nerve Growth Factor (NGF) on the surface of the electrodes.
49. The retinal color prosthesis as in paragraph 1 further comprising a coating of neurotrophic factor on the surface of the insulator near the electrodes.
50. The retinal color prosthesis as in paragraph 49 further comprising a coating of Nerve Growth Factor (NGF) on the surface of the insulator near the electrodes.
51. The retinal color prosthesis as in paragraph 1 further comprising said plurality of electrodes partially insulated.
52. The retinal color prosthesis as in paragraph 51 further comprising electrodes recessed within an insulator.
53. The retinal color prosthesis as in paragraph 51 wherein the electrode surfaces are flat.
54. The retinal color prosthesis as in paragraph 51 wherein the insulating surfaces form a curved surface such as will conform to the curvature of the retina at its designated placement point.
55. The retinal color prosthesis as in paragraph 51 wherein the electrodes are recessed within the insulator, but the sides of the electrodes are exposed.
56. The retinal color prosthesis as in paragraph 1 wherein said electrodes are elongated electrodes, from 100 microns to 500 microns in length.
57. The retinal color prosthesis as in paragraph 1 wherein the electrodes are iridium slugs attached to a substrate by conductive adhesive.
58. The retinal color prosthesis as in paragraph 57 wherein the substrate is covered with aluminum or zirconium oxide or silicone as an insulator with wells where the iridium slugs are located.
59. The retinal color prosthesis as in paragraph 58 wherein the iridium slugs are situated with the insulating wells, fitting against the walls or the well.
60. The retinal color prosthesis as in paragraph 58 wherein the iridium slugs are situated with the insulating wells recessed approximately symmetrically from the sides of the wells.
61. The retinal color prosthesis as in paragraph 60 wherein the iridium slugs are situated within said openings in said covering material; and wherein said slugs are fitted against the sides of the wells.
62. The retinal color prosthesis as in paragraph 60 wherein the iridium slugs are situated within said openings in said covering material; wherein said slugs are recessed symmetrically from the walls of said openings.
63. The retinal color prosthesis as in paragraph 62 wherein the iridium slugs are differently situated relative to the tops of the wells, standing flush with the full height of the wells or recessed below the well tops; the depth of the well ranges from 0.1llm to 1 mm.
64. The retinal color prosthesis as in paragraph 1 further comprising a iridium electrode which is attached onto a foil selected from the group consisting of platinum foil or iridium foil that acts to hermetically seal the area it covers; said foil glued to an aluminum pad with electrically conductive glue; said aluminum pad deposited on a silicon substrate which may also support electronic circuitry; a titanium ring, sputtered, plated, ion implanted, ion-beam assisted deposited (IBAD) or otherwise attached to the platinum or iridium foil; with an insulating layer adhered to the titanium ring; wherein the insulation material is selected from the group consisting of silicon carbide, diamond-like coating, silicon nitride and silicon oxide in combination, titanium oxide, tantalum oxide, aluminum nitride, aluminum oxide and zirconium oxide.
65. The retinal color prosthesis as in paragraph 1 further comprising a titanium nitride electrode which is sputtered onto a platinum or iridium foil that acts to hermetically seal the area it covers, said foil glued to an aluminum pad with electrically conductive glue, said aluminum pad deposited on a silicon substrate which may also support electronic circuitry, a titanium ring, sputtered, plated, ion implanted, or ion-beam assisted deposited (IBAD) to attach it to the platinum or iridium foil, with an insulating layer adhered to the titanium ring; wherein the insulation material is selected from the group consisting of siliconnitride/silicon oxide sandwich, silicon carbide, diamond-like coating, silicon nitride, silicon oxide, silicone, zirconium oxide, silicone, parylene, PTFE (polytetrafluoroethylene) and FEP (fluorinated ethylene propylene).
66. The retinal color prosthesis as in paragraph 64 or paragraph 65 wherein an platinum layer is plated onto the exposed part of the titanium ring and plated onto the layer of a substance selected from the group consisting of silicon carbide, diamondlike coating, silicon nitride and silicon oxide in combination, titanium oxide, tantalum oxide, aluminum nitride, aluminum oxide and zirconium oxide, the platinum layer acting as an electrical conductor.
67. The retinal color prosthesis as in paragraph 65 wherein the platinum electrode is internal to the well formed by a substance selected from the group consisting of silicon carbide, diamond-like coating, silicon nitride and silicon oxide in combination, titanium oxide, tantalum oxide, aluminum nitride, aluminum oxide, zirconium oxide, PTFE (polytetrafluoroethylene). FEP (fluorinated ethylene propylene and waxes, and its titanium ring; said electrode whole angle at it's peak being in the range from1 to 120 ; the base of said conical or pyramidal electrode ranging from 1pm to 500pm; the linear section of the well unoccupied by said conical or pyramidal electrode ranging from zero to one-third.
68. The retinal color prosthesis as in paragraph 65 wherein the platinum electrode has a mushroom-like shape with a narrower stalk and a larger head which has a larger plan view area than the well from which it arises.
69. The retinal color prosthesis as in paragraph 1 wherein hermetic sealing a component of said retinal color prosthesis is accomplished by coating the object to be hermetically sealed selected from the group consisting of silicon carbide, diamondlike coating, silicon nitride and silicon oxide in combination, titanium oxide, tantalum oxide, aluminum nitride, aluminum oxide, zirconium oxide, PTFE (polytetrafluoroethylene), FEP (fluorinated ethylene propylene and waxes.
70. The retinal color prosthesis as in paragraph 1 wherein hermetic sealing of a component of said retinal prosthesis is accomplished by placing it in a metal and ceramic box of rectangular cross-section with the top side and bottom side initially open, the bottom being a material selected from the group consisting of aluminum oxide or zirconium oxide; the top and four sides being a metal selected from the group consisting of platinum, iridium, gold, and stainless steel.
71. The retinal color prosthesis as in paragraph 70 further comprising solder balls placed on the"flip chip", which electrically contact metal feedthroughs made of a metal selected from the group iridium, platinum, titanium, palladium, gold, and stainless steel.
72. The retinal color prosthesis as in paragraph 71 wherein the metalceramic case empty of electronic circuitry, the ceramic base attachment to the metal sides is by brazing; with the flip-chip circuitry inside the metal-ceramic case, the metal top stands attached to the metal sides by laser welding.
73. An eye-motion compensation system comprising
   a. an eye-movement tracking apparatus;
   b. said eye-movement-tracking system measuring eye position, and time;
   c. wherein said measurements are converted to information signals by the eye-movement tracking apparatus;
   d. wherein said information signals are chosen from the group consisting of electromagnetic signals, acoustical signals, and light signals;
   e. wherein said information signals are transmitted by a transmitter on the eye-movement tracking apparatus to a receiver on the video data processing unit;
   f. said video data processor unit interprets eye movement measurements over time as angular positions, angular velocities, and angular accelerations;
   g. said eye position, velocity, acceleration data is further processed by the video data processing unit; wherein feedback information is provided to the video data processor; wherein compensation, stabilization and adjustment for the motion of the eye is provided to an electronic image, from an imager carried by a patient; wherein said image is presentable to the retina by way of an internal-to-the-eye implant.
74. A head motion compensation system comprising a head motion tracking system wherein the motion and position of the head is sensed by a basic sensor selected from the group consisting of an integrating accelerometer, a micro-machined mechanical gyroscope, a laser gyroscope, a combination of an integrating accelerometer and a micro-machined mechanical gyroscope; a combination of an integrating accelerometer and a laser gyroscope; the motion and position of the head is determined by said basic sensor; wherein the data are communicated from the head tracking system to the video data processing unit by telemetry; wherein the data are processed in the video data processor; and wherein said video data processing unit can process the data of the motion of the eye as well as that of the head to further adjust the image electronically whereby the electronic data image is presented to the patient adjusted for eye motion and head motion.
75. A physician's control unit comprising a unit located external to the eye of a patient; a first transceiver located in the physician's control unit; a second transceiver located in the internal-to-the-eye of the patient retinal color prosthesis implant; said physician's control unit transceiver transmits information which controls the image data parameters of the image supplied by external imager; and wherein the physician's control unit receives information which said implanted inside the patient's eye transmits out of the eye.
76. The apparatus of paragraph 75 further comprising said physician's control unit wherein the physician sets control parameters of the image data signal such as maximum and minimum amplitudes, range of pulse widths, range of frequencies, and electrode patterns of electrical stimulation.
77. The apparatus of paragraph 76 wherein said physician's control unit receives and monitors said transmitted-out, of the patient's eye, information including electrode current, electrode impedance, compliance voltage, and electrical recordings from the retina.
78. A physician's hand-held or palm-size test unit comprising a handheld computer used to set up and evaluate a retinal color prosthesis implant during or soon after implantation at the patient's bedside; a transceiver in the hand-held test unit; a transceiver in the retinal color prosthesis internal-to-the-eye implant; said hand-held test unit having the capability of receiving signals which are transmitted out of the eye of the patient and having the ability to send information in to the retinal implant electronic chip; said hand-held computer able to adjust the amplitudes on each electrode, one at a time, or in groups.
79. A patient's control unit comprising a unit located external to the patient's eye and normally carried by the patient, including but not limited to, on a belt, that has patient accessible controls; said controls controlling apparent brightness, apparent contrast, and apparent magnification as presented to the patient's perception by the retinal color prosthesis; said patient's control unit having electronic circuits and components; wherein upon remote command, will respond in a manner similar to that of said physician's local unit; and which has a transmitter and a receiver for remote communication.
80. An electrode apparatus comprising a plurality of electrodes hermetically sealed by a coating of hermetic sealant; wherein said hermetic coating sealant is made from a material selected from the group consisting of silicon carbide, diamond-like coating, silicon nitride and silicon oxide in combination, titanium oxide, tantalum oxide, aluminum nitride, aluminum oxide or zirconium oxide.
81. The apparatus of paragraph 80 further comprising said electrodes from a material selected from the group consisting of pyrolytic carbon, titanium nitride, platinum, iridium, and iridium oxide.
82. The apparatus of paragraph 80 further comprising said plurality of electrodes insulated along their extension, up to their tips.
83. The apparatus of paragraph 80 further comprising said insulation selected from the group consisting of silicon carbide, diamond-like coating, silicon nitride and silicon oxide in combination, titanium oxide, tantalum oxide, aluminum nitride, aluminum oxide, zirconium oxide, PTFE (polytetrafluoroethylene), FEP (fluorinated ethylene propylene and waxes.
84. The apparatus of paragraph 80 further comprising said plurality of electrodes partially insulated.
85. The apparatus of paragraph 84 further comprising electrodes recessed within an insulator.
86. The apparatus of paragraph 85 wherein the electrode surfaces are flat.
87. The apparatus of paragraph 86 wherein the insulating surfaces form a curved surface such as will conform to the curvature of the retina at its designated placement point.
88. The apparatus of paragraph 85 wherein the electrodes are recessed within the insulator, but the sides of the electrodes are exposed.
89. The apparatus in paragraph 80 wherein said electrodes are capacitive electrodes.
90. The apparatus in paragraph 89 wherein said capacitive electrodes are selected as a pair from the group of pairs consisting of iridium and iridium oxide, and, titanium and titanium nitride.
91. The apparatus of paragraph 80 wherein said electrodes are elongated electrodes, from 100 microns to 500 microns in length.
92. The apparatus of paragraph 84 wherein said plurality of electrodes are, being of relative positive polarity, arranged in a unipolar arrangement, with the relative ground electrode on the insulated back of the electrode assembly.
93. The apparatus in paragraph 84 further comprising said plurality of electrodes in a bipolar arrangement.
94. The apparatus in paragraph 84 further comprising said plurality of electrodes in a multipolar arrangement.
95. The apparatus of paragraph 94 further comprising said plurality of electrodes in an electric field focusing arrangement.
96. The apparatus of paragraph 84 further comprising a coating of a neurotrophic factor on the surface of the electrodes.
97. The apparatus as in paragraph 96 further comprising a coating of Nerve Growth Factor (NGF) on the surface of the electrodes.
98. The apparatus of paragraph 84 further comprising a coating of a neurotrophic factor on the surface of the insulator near the electrodes.
99. The apparatus as in paragraph 98 further comprising a coating of Nerve Growth Factor (NGF) on the surface of the insulator near the electrodes.
100. A retinal electrode array coating comprising a coating of neurotrophic factor on the surface of the electrodes.
101. The retinal electrode array coating as in paragraph 100 further comprising a coating of Nerve Growth Factor (NGF) on the surface of the electrodes.
102. The retinal electrode array coating as in paragraph 100 further comprising a coating of neurotrophic factor on the surface of the insulator near the electrodes.
103. The retinal electrode array coating as in paragraph 100 further comprising a coating of Nerve Growth Factor (NGF) on the surface of the insulator near the electrodes.
104. An eye-implantable electronic circuit subsystem comprising one or more electronic circuits hermetically sealed wherein said hermetic sealant is made from a coating selected from the group con tantalum oxide, aluminum nitride, aluminum oxide and zirconium oxide; and wherein said sealant is applied to an eye-implantable electronic circuit subsystem by ion-beam assisted deposition (IBAD).
106. The eye-implantable electronic circuit subsystem of paragraph 104 further comprising said internal component having a configuration of two or more physically separate parts wherein insulated wires join said parts, wherein at least one of said two or more physically separate parts of the internal component is configured as an electronic component and at least one of the other of said two or more separate parts of the internal component is configured as an electrode component.
107. The eye-implantable electronic circuit subsystem of paragraph 106 further comprising: a. an at least one electrode component at least partially implanted subretinally; and b. an at least one electronic component at least partially implanted epiretinally.
108. The eye-implantable electronic circuit subsystem of paragraph 104 further comprising:
   a. an at least one electrode component implanted epiretinally; and
   b. an at least one electronic component fastened subretinally.
109. The eye-implantable electronic circuit subsystem of paragraph 104 further comprising at least one said electronics component, in a position distant from at least one said electrode component, both said components within the eye.
110. The eye-implantable electronic circuit subsystem of paragraph 109 further comprising an insulated conducting coil attached to one electronic component by both ends of said coil.
111. The eye-implantable electronic circuit subsystem of paragraph110 further comprising said coil attached to said electronic component, each end of the insulated conducting coil attached to an electrically different attachment point on said electronic component.
112. The eye-implantable electronic circuit subsystem of paragraph 104 further comprising a plurality of said insulated conducting coils attached to at least one electronic component by both ends of said coil.
113. The eye-implantable electronic circuit subsystem of paragraph 112 further comprising a plurality of said insulated conducting coils attached to at least one said electronic component, each end of the insulated conducting coil attached to an electrically different attachment point on said electronic component.
114. The eye-implantable electronic circuit subsystem of paragraph 113 further comprising an insulated conducting coil attached to one electrode component by both ends of said coil.
115. The eye-implantable electronic circuit subsystem of paragraph 114 further comprising said coil attached to said electrode component, each end of the insulated conducting coil attached to an electrically different attachment point on said electrode component.
116. The eye-implantable electronic circuit subsystem of paragraph 115 further comprising a plurality of said insulated conducting coils attached to at least one of said electrode components by both ends of said coil.
117. The eye-implantable electronic circuit subsystem of paragraph 115 further comprising a plurality of said coils attached to said electrode component, each end of said insulated conducting coil attached to an electrically different attachment point on said electrode component.
118. An eye-implantable subsystem comprising a configuration of two physically separate parts wherein insulated wires join said parts.
119. The eye-implantable subsystem as in paragraph 118 further comprising one of said two separate parts of the internal component that is configured as a combined electronic and electrode component and the other of said two separate parts of the internal component which is also configured as a combined electronic and electrode component.
120. The eye-implantable subsystem as in paragraph 119 further comprising one of said combined electronic and electrode components subretinally positioned and the other of said combined electronic and electrode components epiretinally positioned so as to efficiently stimulate bipolar and associated cells in the retina.
121. The eye-implantable subsystem as in paragraph 120 further comprising one of said two physically separate parts of the eye-implantable subsystem that is configured as an electronic component; and wherein the other of said two physically separate parts of the eye-implantable subsystem is configured as an electrode component.
122. The eye-implantable subsystem as in paragraph 121 further comprising at least one electrode component subretinally positioned and at least one electronic component epiretinally positioned.
123. The eye-implantable subsystem as in paragraph 122 further comprising at least one electronics component in a position distant, within the eye, from said electrode component; wherein said electrode component is placed near the retina; whereby the relatively high heat dissipation of electronic component has a minimal effect on the heat sensitive retina.
124. The eye-implantable subsystem as in paragraph 123 further comprising at least one electronics component in a position centrally within the vitreous cavity.
125. An eye-implantable subsystem forinductively transferring electromagnetic energy into and out from an electronic component located internally in the eye, comprising:
   a. a coil external to a patient ;
   b. two insulated conducting coils, both located within the ocular orbit;
   c. one free end of a first coil joined to one free end of a second coil;
   d. a second free end of said first coil joined to a second free end of said second coil;
   e. a third coil attached integrally to an internal electronic component located within the eye;
   f. said second coil located in proximity to said third coil.
   g. wherein said first coil is inductively coupled by a changing electromagnetic field in the coil external to the patient; wherein said first coil is electrically connected to the second coil whereby the current in the second coil conforms to the current in the first coil; wherein said second coil is inductively coupled to said third coil by a second changing electromagnetic field; whereby a current produced in the third coil supplies energy to the electronic component located within the eye;
   h. wherein a changing current supplied to the third coil by the electronic component within the eye produces a changing electromagnetic field in said third coil; wherein said third coil is inductively coupled to the second coil; wherein said second coil is electrically connected to said first coil; whereby a changing electromagnetic field is produced by the first coil;
   i. wherein said first coil produces a changing electromagnetic field which can inductively couple to said coil external to the patient.
126. The eye-implantable subsystem of paragraph 125 wherein the position of said second coil is toward the lens of the eye; and said first coil is toward the front of the eye.
127. The eye-implantable subsystem of paragraph 126 wherein said second coil is positioned toward the retina of the eye; and said first coil is positioned toward the front of the eye.
128. An apparatus which transfers acoustic energy and information into and out from an electronic component located internally in the eye, comprising:
   a. a first acoustical bi-directional transducer located within the eye;
   b. wherein said bi-directional transducer acts as a transmitter and a receiver of acoustical energy;
   c. a second bi-directional acoustical transducer located on an external unit worn on the head;
   d. wherein said bi-directional transducer acts as a transmitter and a receiver of acoustical energy;
   e. acoustic signal information and acoustic energy transmitted from one of said transducers is received by other of said transducers;
   f. each of said acoustical transducers receives and amplifies a received signal and received power;
   g. each of said acoustical transducers converts the received signal and the received power to an electrical signal and to electrical power.
129. A method for making a retinal prosthetic for color sight restoration comprising the stepsof
   a. imaging with a color imager to capture color image, said color imaging being part of an external component, which is carried by the patient;
   b. processing video data with a video data processing unit for converting said image to electrical signals which contain image color signal data, said color imaging being part of said external component, which is carried by the patient;
   c. implanting internal to the eye a component which has a plurality of electrodes and one or more electronic circuits;
   d. communicating between the external receiver and transmitter and the internal receiver and transmitter for communication between the external component and the internal component;
   e. configuring said electronic circuits to receive color signal information from the video data processing unit, activate the electrodes based on said color signal information and extract electrical power from the received signal;
   f. configuring said electrodes to stimulate bipolar cells of the retina electrically.
130. The method as in paragraph 129 further comprising the step of processing data with a video data processing unit which converts said color image to electrical signals corresponding to data for a pixel or grid-like arrangement of electrodes placed within the eye.
131. The method as in paragraph 129 further comprising the step of compensating for eye-motion with an eye-motion compensation system.
132. The method as in paragraph 131 comprising the stepsof :
   a. tracking eye-movements;
   b. measuring eye movements;
   c. transmitting said measurements to video data processor unit; interpreting eye movement measurements as angular positions, angular velocities, and angular accelerations;
   d. processing eye position, velocity, acceleration data by the video data processing unit;
   e. compensating, stabilizing and adjusting image electronically.
133. The method as in paragraph 132 further comprising the steps of, for a head tracking system, determining the motion and position of the head by an apparatus selected from the group consisting of an inertial unit with an integrating accelerometer, a laser gyroscope, and a combined inertial unit (with an integrating accelerometer) together with a laser gyroscope; processing the data in the video data processor; communicating the data from the head tracking system to the video data processing unit by telemetry.
134. The method as in paragraph 133 further comprising the steps of processing video data image and other data related to said image signal data, including electronic compensation for the motion of the eye (s) and including electronic compensation for the motion of the head; utilizing said video data processing unit.
135. The method as in paragraph 129 further comprising the steps of monitoring and adjusting, upon external command, data to and from the internal retinal implants using programmable external and internal units.
136. The method as in paragraph 129 further comprising the steps of, for a physician's control unit, locating a unit external to the eye; controlling information supplied by external image signal data at physician's choice; and receiving diagnostic which an implanted unit inside the eye transmits out of the eye.
137. The method as in paragraph 136 further comprising the steps of controlling and setting the parameters, by the physician, of the image data signal such as amplitudes, pulse widths, frequencies, and patterns of electrical stimulation.
138. The method as in paragraph 136 further comprising the steps of receiving and monitoring said transmitted-out information including electrode current, electrode impedance, compliance voltage, and electrical recordings from the retina, utilizing the physician's control unit.
139. The method as in paragraph 129 further comprising the steps of, for a physician's hand-held unit, setting up and evaluating a retinal color prosthesis implant soon after the implantation, at the patient's bedside utilizing a handheld computerbased unit; receiving signals transmitted out of the eye; transmitting information in to the retinal color implant electronic chip; adjusting the amplitudes on each electrode, one at a time, or in groups; determining success of the retinal implant.
140. The method as in paragraph 129 further comprising the steps of, for a patient's control unit, controlling apparent brightness, contrast, and magnification utilizing a unit external to the eye; managing the controls by the patient.
141. The method as in paragraph 130 further comprising the steps of encoding and transmitting time sequences and widths of pulses for signaling color to bipolar cells, using said video data processing unit.
142. The method as in paragraph 130 further comprising the stepsof : processing video data; encoding electrical signal information for signaling color to the different bipolar cells utilizing the video data processor, in a planar pattern, corresponding to the location of electrodes in the vicinity of the individual bipolar cells, including red-center-green surround, green-center-red-surround, blue-centeryellow surround and yellow-center-blue-surround bipolar cells.
143. The method as in paragraph 129 further comprising the steps of hermetically sealing and partially insulating one or more electrode subpart (s).
144. The method as in paragraph 129 further comprising the step of hermetically sealing one or more electronic circuit subpart (s).
145. The method as in paragraph 129 further comprising the step of transferring received processed image data to the electrodes by utilizing electronic circuits within the eye.
146. The method as in paragraph 145 further comprising the step of applying received color signal information to a plurality of electrodes, corresponding to the original external pixel-like scene capture; utilizing electronic circuits.
147. The method as in paragraph 129 further comprising the steps of measuring and transmitting out of the eye the signal power level being received internally; utilizing electronic circuits.
148. The method as in paragraph 129 further comprising the steps of measuring and transmitting out of the eye an impedance for each of the different electrodes; utilizing electronic circuits.
149. The method as in paragraph 129 further comprising the steps of measuringelectrophysiologic retinal recordings and transmitting them out of the eye; utilizing electronic circuits.
150. The method as in paragraph 129 further comprising the step of implanting fully subretinally said internal component.
151. The method as in paragraph 129 further comprising the step of implanting epiretinally said internal component.
152. The method as in paragraph 129 further comprising the step of configuring said plurality of electrodes in a monopolar arrangement.
153. The method as in paragraph 129 further comprising the step of configuring said plurality of electrodes in a bipolar arrangement.
154. The method as in paragraph 129 further comprising the step of configuring said plurality of electrodes in a multipolar arrangement.
155. The method as in paragraph 155 further comprising the step of configuring said plurality of electrodes in an electric field focusing arrangement.
156. The method as in paragraph 129 further comprising the step of selecting said plurality of electrodes comprises a substance from the group consisting of pyrolytic carbon, titanium nitride, platinum, iridium, and iridium oxide.
157. The method as in paragraph 143 or paragraph 144 further comprising the step of selecting an hermetic sealant coating substance from the group consisting of silicon carbide, diamond-like coating, silicon nitride and silicon oxide in combination, titanium oxide, tantalum oxide, aluminum nitride, aluminum oxide, zirconium oxide, PTFE (polytetrafluoroethylene), FEP (fluorinated ethylene propylene and waxes.
158. The method as in paragraph 129 further comprising the step of insulating said plurality of electrodes along their extension, up to their tips.
159. The method as in paragraph 158 further comprising the step of selecting said insulation from the group consisting of silicon carbide, diamond-like coating, silicon nitride and silicon oxide in combination, titanium oxide, tantalum oxide, aluminum nitride, aluminum oxide, zirconium oxide, PTFE (polytetrafluoroethylene), FEP (fluorinated ethylene propylene and waxes.
160. The method as in paragraph 129 further comprising the step of selecting a pair of substances to form a plurality of capacitive electrodes, the group of pairs consisting of the pairs iridium and iridium oxide, and, titanium and titanium nitride.
161. The method as in paragraph 129 further comprising the step of mounting a plurality of capacitors on the electrode substrate in a one-to-one correspondence with the plurality of electrodes.
162. The method as in paragraph 129 further comprising the step of configuring said internal component into two separate parts wherein insulated wires join said parts.
163. The method as in paragraph 162 further comprising the steps of configuring one of said two separate parts of the internal component as a combined electronic and electrode component and configuring the other of said two separate parts of the internal component as a combined electronic and electrode component.
164. The method as in paragraph 163 further comprising the steps of positioning one of said combined electronic and electrode component subretinally and positioning the other of said combined electronic and electrode component epiretinally.
165. The method as in paragraph 164 further comprising the step of holding said two components positioned in a prescribed relationship to each other by small magnets.
166. The method as in paragraph 164 further comprising the step of holding said two components in a prescribed relationship to each other by alignment pins.
167. The method as in paragraph 164 further comprising the step of holding said two components positioned in a prescribed relationship to each other by snap-together mating parts.
168. The method as in paragraph 162 further comprising the steps of configuring one of said two separate parts of the internal component as an electronic component and configuring the other of said two separate parts of the internal component as an electrode component.
169. The method as in paragraph 168 further comprising the steps of positioning said electrode component subretinally and said positioning said electronic component epiretinally.
170. The method as in paragraph 169 further comprising the steps of placing said electrode component near the retina; and placing said electronics component in a position distant, within the eye, from said electrode component.
171. The method as in paragraph 129 further comprising the steps of attaching an insulated conducting coil to the internal component, attaching each end of the coil to an electrically different attachment point on said internal component.
172. The method as in paragraph 171 further comprising the steps of joining two insulated coils, the first larger, and the second smaller, joining one free end of the first coil to one free end of the second coil, joining the other free end of said first coil to the other free end of said second coil, placing said smaller coil in proximity to a coil attached to said internal , component, positioning and fastening said larger coil toward the lens of the eye.
173. The method as in paragraph 129 further comprising the steps of building an internal component in two parts, the first part an electronics part, the second part an electronics and electrode part; integrating an insulated conducting pick-up coil into each of the first and second internal component parts; joining two insulated conducting coils, one larger, and the second smaller, joining one free end of the first larger coil to one free end of the second smaller coil, joining the other free end of said first larger coil to the other free end of said second smaller coil, attaching said second smaller coil in proximity to said coil integrated to the first internal part ; locating said first internal part in the fatty tissue behind the eye; locating the second internal part within the eye; attaching said larger coil to the temporal region of the head, in the vicinity of the eye.
174. The method as in paragraph 129 further comprising the step of coating a neurotrophic factor onto the surface of the electrodes.
175. The method as in paragraph 174 further comprising the step of coating a Nerve Growth Factor (NGF) on the surface of the electrodes.
176. The method as in paragraph 129 further comprising the step of coating a neurotrophic factor on the surface of the insulator near the electrodes.
177. The method as in paragraph 176 further comprising the step of coating a Nerve Growth Factor (NGF) on the surface of the insulator near the electrodes.
178. The method as in paragraph 129 further comprising the step of partially insulating said plurality of electrodes.
179. The method as in paragraph 178 further comprising the step of recessing electrodes within an insulator.
180. The method as in paragraph 179 further comprising the step of forming the electrodes with flat surfaces.
181. The method as in paragraph 179 further comprising the step of forming the insulating surfaces as a curved surface;conforming said curvature to the curvature of the retina at its designated placement point.
182. The method as in paragraph 179 further comprising the steps of recessing the electrodes within the insulator; and exposing the sides of the electrodes.
183. The method as in paragraph 129 further comprising the step of fabricating elongated electrodes from 100 microns to 500 microns in length.
184. The method as in paragraph 129 further comprising the step of attaching iridium slug electrodes to a substrate by conductive adhesive.
185. The method as in paragraph 184 further comprising the step of covering the substrate with aluminum or zirconium oxide or silicone; leaving openings where the iridium slugs are located.
186. The method as in paragraph 185 further comprising the steps of situating the iridium slugs, one-to-one, within said openings, in said covering material; fitting said iridium slugs against the walls of said openings.
187. The method as in paragraph 185 further comprising the steps of situating the iridium slugs, one-to-one, within said openings in said covering material; and recessing said iridium slugs symmetrically from the walls of said openings.
188. The method as in paragraph 186 or in paragraph 187 further comprising the step of situating said iridium slugs to stand with their tops flush with the tops of the openings where the depth of the openings range from 0.1 pm to 1 mm.
189. The method as in paragraph 186 or in paragraph 187 further comprising the step of situating said iridium slugs to stand with their tops recessed below the top of the openings where the depth of the openings range from 0.1 pm to 1 mm.
190. The method as in paragraph 189 further comprising the step of constructing a iridium electrode assembly, further comprising the steps of depositing an aluminum pad on a silicon substrate, which may also support electronic circuitry; electroplating iridium onto a foil selected from the group consisting of platinum or iridium foil; sealing the area said foil covers hermetically by said foil, gluing said foil to said aluminum pad with electrically conductive glue; attaching, sputtering, plating, ion implanting, or ion-beam assisted depositing (IBAD) a titanium ring, to the platinum or iridium foil; adhering an insulating layer to the titanium ring, where insulation material is selected from the group consisting of silicon carbide, diamondlike coating, silicon nitride and silicon oxide in combination, titanium oxide, tantalum oxide, aluminum nitride, aluminum oxide and zirconium oxide.
191. The method as in paragraph 129 further comprising the step of constructing a titanium electrode assembly, further comprising the steps of depositing an aluminum pad on a silicon substrate, which may also support electronic circuitry; electroplating titanium onto a platinum or iridium foil; sealing the area said foil covers hermetically by said foil, gluing said foil to said aluminum pad with electrically conductive glue; attaching, sputtering, plating, ion implanting, ion-beam assisted depositing (IBAD) a titanium ring, to the platinum or iridium foil; adhering an insulating layer to the titanium ring, where insulation material is selected from the group consisting of silicon carbide, diamond-like coating, silicon nitride and silicon oxide in combination, titanium oxide, tantalum oxide, aluminum nitride, aluminum oxide and zirconium oxide.
192. The method as in paragraph 190 or paragraph 191 further comprising the step of plating an aluminum layer onto the exposed part of the titanium ring and onto a material selected from the group consisting of silicon carbide, diamond-like coating, silicon nitride and silicon oxide in combination, titanium oxide, tantalum oxide, aluminum nitride, aluminum oxide and zirconium oxide, said aluminum layer acting as an electrical conductor.
193. The method as in paragraph 191 further comprising the steps of forming said platinum electrode internal to the well formed by a substance selected from the group consisting of silicon carbide, diamond-like coating, silicon nitride and silicon oxide in combination, titanium oxide, tantalum oxide, aluminum nitride, aluminum oxide and zirconium oxide, and its titanium ring; fabricating said electrode with its whole peak angle in the range from 1 to 120 ; forming the base of said conical or pyramidal electrode in the range from 1m to 500J. m; forming the linear section of the well unoccupied by said conical or pyramidal electrode in the range ranging from zero to one-third of the whole linear section.
194. The method as in paragraph 191 further comprising the steps of forming the platinum electrode with a mushroom-like shape with a narrower stalk; constructing said electrode head with a larger plan view area than the well from which it arises.
195. The method as in paragraph 129 further comprising the step of hermetically sealing a component of said retinal color prosthesis by coating said component with a substance selected from the group consisting of silicon carbide, diamond-like coating, silicon nitride and silicon oxide in combination, titanium oxide, tantalum oxide, aluminum nitride, aluminum oxide, zirconium oxide, PTFE (polytetrafluoroethylene), FEP (fluorinated ethylene propylene and waxes.
196. The method as in paragraph 129 further comprising the step of hermetic sealing a component of said retinal color prosthesis by placing it in a metal and ceramic box of rectangular cross-section with the top side and bottom side initially open, the bottom being a ceramic selected from the group consisting of aluminum oxide and zirconium oxide; the top and four sides being a metal selected from the group consisting of platinum, iridium, palladium, gold, and stainless steel.
197. The method as in paragraph 196 further comprising the steps of placing solder balls on a"flipchip" ; selecting a metal for the metal feedthroughs which the contact the solder balls from the group iridium, platinum, titanium, palladium, gold, and stainless steel; and plating the inner surface of the feed-through, toward the solder ball being plated with gold or with nickel.
198. The method as in paragraph 197 further comprising the steps of brazing the ceramic base of the metal-ceramic case to the metal sides of said case with said metal-ceramic case empty of electronic circuitry; laser welding, subsequently, with the flip chip circuitry inside the case, the metal top of said case to the metal sides of said case.
199. The method as in paragraph 129 further comprising the steps of compensating for eye motion further comprising the steps of measuring eye motion; transmitting said eye motion information to said video data processing unit; interpreting eye movements data as angular positions, angular velocities, and angular acceleration; processing of eye position motion data by the video data processing unit; compensating for eye movement effects on the image by electronic compensation, stabilization and adjustment.
200. The method as in paragraph 129 further comprising the steps of receiving diagnostic information from and supplying calibration and adjustment information to said retinal color prosthetic utilizing a physician's control unit; receiving and transmitting said information with said physicians control unit.
201. The method as in paragraph 129 further comprising the steps of the physician, or a medical technician, controlling and setting parameters of the image data signal such as amplitudes, pulse widths, frequencies, and patterns of electrical stimulation utilizing the physician's control unit.
202. The method as in paragraph 129 further comprising the steps of the physician, or a medical technician, is monitoring transmitted-out information including electrode current, electrode impedance, compliance voltage, and electrical recordings from the retina, utilizing the physician's control unit.
203. The method as in paragraph 129 further comprising the steps of utilizing a physician's post-operative hand held computer-based test unit to set up and to evaluate a retinal prosthesis implant during or soon after implantation, at the patient's bedside; sending information to and receiving information from the video data processing unit utilizing said physician's post-operative unit; sending information to the retinal implant electronic chip; adjusting the amplitudes on each electrode, one at a time, or in groups utilizing said hand-held computer-based test unit; and determining the success of the retinal prosthesis.
204. The method as in paragraph 129 further comprising the steps of controlling apparent brightness, controlling apparent contrast and controlling magnification utilizing a patient-operated patient's control unit; locating said patient's unit external to the eye.
205. The method as in paragraph 129 further comprising the steps of utilizing a physician's remote unit to perform all of the functions of the local physician's control unit; communicating with the video data processing unit, both transmitting set-up and control commands, and receiving diagnostic and monitoring information.
206. A method for making an eye-motion compensation system comprising the stepsof :
   a. tracking eye-movements;
   b. measuring eye movements as to position and time;
   c. converting said measurements to information signals
   d. transmitting said measurements to video data processor unit;
   e. interpreting eye movement measurements as angular positions, angular velocities, and angular accelerations;
   f. processing eye position, velocity, and acceleration data by the video data processing unit;
   g. compensating, stabilizing and adjusting an imager generated image, said imager carried by a patient, wherein an imager generated image, presentable to the retina via an internal-to-the-eye implant, is electronically compensated, stabilized and adjusted for the motion of the eye.
207. The method of paragraph 206 further comprising the stepsof : selecting a head tracking apparatus from the group consisting of an inertial unit with an integrating accelerometer, a laser gyroscope, and a combined inertial unit with an integrating accelerometer together with a laser gyroscope; determining the motion and position of the head by said apparatus; processing the data from said apparatus in the video data processor; transmitting the data from said apparatus the head tracking system to the video data processing unit by telemetry; processing the data of the motion of the eye as well as that of the head; adjusting an imager generated image, presentable to the retina via an internal-to-the-eye implant, electronically; compensating, stabilized and adjusted for the motion of the eye and head.
208. A method for enabling a physician to control and evaluate the parameters for a retinal color prosthesis comprising the steps of controlling information supplied by an external-to-the-patient image signal to an internal-to-theeye implant; and receiving diagnostic information from the implant.
209. The method of paragraph 208 further comprising the steps of the physician controlling and setting the parameters of the image data signal such as amplitudes, pulse widths,
frequencies, and patterns of electrical stimulation. 210. The method of paragraph 209 further comprising the steps of receiving and monitoring information from the internal-to-the-eye implant including electrode current, electrode impedance, compliance voltage, and electrical recordings from the retina.
211. A method for enabling a physician to set up the settable parameters of and evaluate the success of a retinal color prosthesis implant soon after the implantation comprising the steps of utilizing a handheld computer-based unit at the patient's bedside; receiving signals transmitted out of the eye from an internal-tothe-eye retinal color prosthesis implant; transmitting information in to the retinal color implant electronic chip component; adjusting the amplitudes on each electrode, one at a time, or in groups.
212. A method for enabling a patient to control some parameters of a retinal color prosthesis comprising the steps of controlling apparent brightness, contrast and magnification of the patient's perception; utilizing an imager to supply an electronic image signal to a video data processing unit; utilizing a control unit external to the eye; plugging said unit external to the eye into a mating plug on the video data processing unit of said retinal processing unit; controlling patient settable parameters by patient operated controls; resetting the video data processing unit parameters; keeping the reset parameter settings until reset by the patient or a physician.
213. A method for making an implantable, retinal-cell stimulating electrode array comprising the step of selecting a plurality of electrodes from the group consisting of pyrolytic carbon, titanium nitride, platinum, iridium, and iridium oxide.
214. The method of paragraph 213 further comprising the steps of hermetic sealing said electrode array by coating said electrode array with a material selected from the group consisting of silicon carbide, diamond-like coating, silicon nitride and silicon oxide in combination, titanium oxide, tantalum oxide, aluminum nitride, aluminum oxide, zirconium oxide, PTFE (polytetrafluoroethylene), FEP (fluorinated ethylene propylene and waxes..
215. The method of paragraph 213 further comprising the step of insulating said plurality of electrodes along their extension, up to their tips.
216. The method of paragraph 215 further comprising the step of selecting said insulation from the group consisting of silicon carbide, diamond-like coating, silicon nitride and silicon oxide in combination, titanium oxide, tantalum oxide, aluminum nitride, aluminum oxide, zirconium oxide, PTFE (polytetrafluoroethylene), FEP (fluorinated ethylene propylene and waxes.
217. The method of paragraph 213 further comprising the step of partially insulating said plurality of electrodes.
218. The method of paragraph 213 further comprising the step of recessing electrodes within an insulator.
219. The method of paragraph 218 further comprising the step of constructing the electrode surfaces as flat surfaces flush with the bottom of the insulator recess.
220. The method of paragraph 219 further comprising the step of conforming the curvature of the insulating surfaces to the curvature of the retina at the designated placement area of the insulating surface.
221. The method of paragraph 218 further comprising the steps of recessing the electrodes within the insulator; and exposing the sides of the electrodes.
222. The method of paragraph 213 further comprising the step of constructing said electrodes as capacitive electrodes.
223. The method of paragraph 222 further comprising the step of selecting said capacitive electrodes as a pair from the group of pairs consisting of iridium and iridium oxide, and, titanium and titanium nitride.
224. The method of paragraph 213 further comprising the step of producing said electrodes as elongated electrodes, from 100 microns to 500 microns in length.
225. The method of paragraph 213 further comprising the step of arranging said plurality of electrodes, in a unipolar arrangement, with the indifferent electrode on the insulated back of the electrode assembly.
226. The method of paragraph 213 further comprising the step of arranging said plurality of electrodes in a bipolar arrangement.
227. The method of paragraph 213 further comprising the step of arranging said plurality of electrodes in a multipolar arrangement.
228. The method of paragraph 227 further comprising the step of arranging said plurality of electrodes in an electric field focusing arrangement.
229. The method of paragraph 213 further comprising the step of coating a neurotrophic factor on the surface of the electrodes.
230. The method of paragraph 229 further comprising the step of coating a Nerve Growth Factor (NGF) on the surface of the electrodes.
231. The method of paragraph 213 further comprising the step of coating a neurotrophic factor on the surface of the insulator near the electrodes.
232. The method of paragraph 231 further comprising the step of coating a Nerve Growth Factor (NGF) on the surface of the insulator near the electrodes.
233. A method for a neurotrophic coating comprising the step of coating an electrode array with a neurotrophic factor on the surface of the electrodes.
234. The method for a neurotrophic coating of paragraph 233 further comprising the step of coating Nerve Growth Factor (NGF) on the surface of the electrodes.
235. The method for a neurotrophic coating of paragraph 233 further comprising the step of coating a neurotrophic factor on the surface of the insulator near the electrodes.
236. The method for a neurotrophic coating of paragraph 23 further comprising the step of coating Nerve Growth Factor (NGF) on the surface of the insulator near the electrodes.
237. A method for making an eye-implantable electronic circuit system comprising the steps of hermetically sealing one or more electronic circuits with a coating selected from the group consisting of silicon carbide, diamond-like coating, silicon nitride and silicon oxide in combination, titanium oxide, tantalum oxide, aluminum nitride, aluminum oxide, zirconium oxide, PTFE (polytetrafluoroethylene), FEP (fluorinated ethylene propylene and waxes.
238. The method of paragraph 237 further comprising the step of configuring said eye-implantable electronic circuit system as two or more separate parts wherein insulated wires join said parts.
239. The method of paragraph 238 further comprising the steps of configuring at least one of said two or more separate parts of the eye-implantable electronic circuit system as a predominantly electronic component and configuring at least one of the other of said two or more separate parts of the eye-implantable electronic circuit system as an electrode component.
240. The method of paragraph 239 further comprising the stepsof :
   a. implanting at least one said electrode component at least partially subretinally; and
   b. implanting at least one said electronic"component at least partially epiretinally.
241. The method of paragraph 239 further comprising the stepsof :
   a. implanting at least one said electrode component; and
   b. implanting at least one said electronic component subretinally.
242. The method of paragraph 241 further comprising at least one said electronics component, in a position distant, in the eye, from at least one said electrode component, in the eye, positioned near the retina.
243. The method of paragraph 241 further comprising the step of positioning at least one electronics component in a position centrally within the vitreous cavity.
244. A method for constructing a coil system for driving a retinal color prosthetic internal-to-the eye electronic implant component comprising the step of attaching an insulated conducting coil to an internal-to-the-eye electronic implant component by both ends of said coil.
245. The method of paragraph 244 further comprising the steps of attaching said coil to an electronic internal component; attaching each end of the insulated conducting coil to an electrically different attachment point on said electronic component.
246. The method of paragraph 244 further comprising the step of attaching a plurality of insulated conducting coils to at least one electronic component by both ends of said coils.
247. The method of paragraph 244 further comprising the steps of attaching a plurality of insulated conducting coils to at least one electronic internal component; attaching each end of said insulated conducting coils to an electrically different attachment point on at least one said electronic internal component.
248. The method for a coil system of paragraph 244 further comprising the step of attaching an insulated conducting coil to an electrode internal component by both ends of said coil.
249. The method of paragraph 248 further comprising the steps of attaching said coil to said electrode internal component; attaching each end of the coil to an electrically different attachment point on said electrode internal component.
250. The method of paragraph 249 further comprising the step of attaching a plurality of said insulated conducting coils to at least one electrode internal component by both ends of said coil.
251. The method of paragraph 250 further comprising the steps of attaching a plurality of said coils to said electrode internal component; attaching each end of the insulated conducting coil to an electrically different attachment point on said electrode internal component.
252. The method of paragraph 244 further comprising the steps:
   a. locating two insulated conducting coils located within the ocular orbit;
   b. joining a first free end of a first coil to a first free end of the second coil;
   c. joining a second free end of said first coil to a second free end of said second coil;
   d. attaching integrally a third coil to an internal electronic part located within the eye;
   e. locating said second coil in proximity to said third coil.
253. The method of paragraph 252 further comprising the steps of positioning said second coil toward the lens of the eye; and positioning said first coil toward the front of the eye.
254. The method of paragraph 253 further comprising the steps of positioning said second coil toward the retina of the eye; and positioning said first coil toward the front of the eye.
255. A method for an acoustical energy and information transfer which transfers acoustic energy and information from and to a retinal color prosthesis internal-to-the-eye electronic implant component located internally in the eye, comprising the stepsof :
   a. locating an acoustical transducer in the eye;
   b. locating an acoustical transducer on an external unit worn on the head;
   c. transmitting acoustic signal information and acoustic energy from the first and from the second of said transducers;
   d. receiving acoustical signal information and acoustical energy by the second and by the first transducers;
   e. amplifying said received acoustical signal information and acoustical power by said acoustical transducers.
256. An apparatus which transfers light energy and information into an electronic component located internally in the eye, comprising:
   a. a photogenerator located externally to the eye; wherein a modulated electrical signal is turned into a modulated light signal;
   b. a photodetector located on the internally located electronic component;
   c. light energy and information transmitted by the photogenerator;
   d. light information and energy received by the photodetector; wherein the modulated light signal is turned into a modulated electrical signal;
   e. an electronic component located internally to the eye; wherein energy and information are extracted from the electrical signal.
257. An apparatus which transfers light energy and information out from an electronic component located internally in the eye, comprising:
   a. a photogenerator located internally to the eye on an electronic component; wherein a modulated electrical signal is turned into a modulated light signal;
   d. a photodetector located externally located externally to the eye;
   e. light energy and information transmitted by the photogenerator;
   f. light information and energy received by the photodetector; wherein the plurality of modulated light signals is turned into a plurality of modulated electrical signals;
   g. at least one electronic circuit located externally to the eye; wherein energy and information are extracted from the electrical signal;
258. An apparatus which transfers light energy and information into and out from an electronic component located internally in the eye, comprising:
   a. a photogenerator located externally to the eye; wherein a modulated electrical signal is turned into a modulated light signal;
   b. a photodetector located on the internally located electronic component;
   c. a photogenerator located internally to the eye on an electronic component; wherein a modulated electrical signal is turned into a modulated light signal;
   d. a photodetector located externally located externally to the eye;
   e. light energy and information transmitted by a plurality of photogenerators;
   f. light information and energy received by a plurality of photodetectors; wherein the plurality of modulated light signals is turned into a plurality of modulated electrical signals;
   h. at least one electronic circuit located externally to the eye; wherein energy and information are extracted from the electrical signal;
   i. an electrical component located internally to the eye; wherein energy and information are extracted from the electrical signal.
   j. said external photogenerator disposed as to send light information and energy to the internal photodetector;
   k. said internal light generator disposed so as to send light information and energy to the external photodetector.
259. A method for light energy and information transfer, which transfers light energy into an electronic component, located internally in the eye, comprising the stepsof :
   a. locating a photogenerator externally to the eye; wherein a modulated electrical signal is turned into a modulated light signal;
   b. locating a photodetector on the internally located electronic component;
   c. transmitting light energy and information from the photogenerator;
   d. receiving light information and energy by the photodetector; wherein the modulated light signal is turned into a modulated electrical signal;
   e. locating an electrical component internally to the eye; extracting energy and information from the electrical signal.
260. A method for light energy and information transfer, which transfers light energy out of an electronic component, located internally in the eye, comprising the stepsof :
   a. locating a photogenerator internally to the eye on an electronic component; turning a modulated electrical signal into a modulated light signal;
   b. locating a photodetector externally to the eye;
   c. transmitting light energy and information from the photogenerator;
   d. receiving light information and energy from the photodetector; turning the modulated light signal into turned a modulated electrical signal; e. locating at least one electronic circuit externally to the eye; extracting energy and information from the electrical signal;
261. A method for light energy and information transfer, which transfers light energy into and out of an electronic component, located internally in the eye, comprising the stepsof :
   a. locating a photogenerator externally to the eye; turning a modulated electrical signal into a modulated light signal;
   b. locating a photodetector on the internally located electronic component;
   c. locating a photogenerator internally to the eye on an electronic component; turning a modulated electrical signal into a modulated light signal;
   d. locating a photodetector externally to the eye;
   e. transmitting light energy and information from a plurality of light generators;
   f. receiving light information and energy by a plurality of photodetectors; turning the plurality of modulated light signals into a plurality of modulated electrical signals;
   g. locating at least one electronic circuit externally to the eye; extracting energy and information from the electrical signal;
   h. locating an electrical component internally to the eye; extracting energy and information from the electrical signal.
   i. disposing said external photogenerator so as to send light information and energy to the internal photodetector;
   j. disposing said internal light generator so as to send light information and energy to the external photodetector.
262. An iridium electrode for a retinal color prosthesis comprising an iridium electrode which is attached onto a foil selected from the group consisting of platinum foil and iridium foil that acts to hermetically seal the area it covers; said foil glued to an aluminum pad with electrically conductive glue; said aluminum pad deposited on a silicon substrate which may also support electronic circuitry; a titanium ring, sputtered, plated, ion implanted, ion beam assisted deposited (27) or otherwise attached to the platinum or iridium foil; with an insulating layer adhered to the titanium ring, where insulation material is selected from the group consisting of silicon carbide, diamond-like coating, silicon nitride and silicon oxide in combination, titanium oxide, tantalum oxide, aluminum nitride, aluminum oxide and zirconium oxide.
263. A titanium nitride electrode for a retinal color prosthesis comprising a titanium nitride electrode which is sputtered onto a foil selected from the group consisting of platinum foil and iridium foil that acts to hermetically seal the area it covers; said foil glued to an aluminum pad with electrically conductive glue; said aluminum pad deposited on a silicon substrate which may also support electronic circuitry; a titanium ring, sputtered, plated, ion implanted, ion-beam assisted deposited (IBAD) or otherwise attached to the platinum or iridium foil; with an insulating layer adhered to the titanium ring, where insulation material is selected from the group silicon carbide, diamond-like coating, silicon nitride and silicon oxide in combination, titanium oxide, tantalum oxide, aluminum nitride, aluminum oxide and zirconium oxide.
264. A method for making an iridium electrode assembly for a retinal color prosthesis comprising the step of constructing an iridium electrode assembly, further comprising the steps of depositing an aluminum pad on a silicon substrate, which may also support electronic circuitry; electroplating iridium onto a foil selected from the group consisting of platinum or iridium foil; sealing the area said foil covers hermetically by said foil, gluing said foil to said aluminum pad with electrically conductive glue; attaching, sputtering, plating, ion implanting, ion-beam assisted depositing (IBAD) a titanium ring, on to the platinum or iridium foil; adhering an insulating layer to the titanium ring, where insulation material is selected from the group consisting of silicon carbide, diamond-like coating, silicon nitride and silicon oxide in combination, titanium oxide, tantalum oxide, aluminum nitride, aluminum oxide and zirconium oxide.
265. A method for making a titanium nitride electrode assembly for a retinal color prosthesis comprising the step of constructing a titanium electrode assembly, further comprising the steps of depositing an aluminum pad on a silicon substrate, which may also support electronic circuitry; electroplating titanium onto a foil selected from the group consisting of platinum foil or iridium foil; sealing the area said foil covers hermetically by said foil; gluing said foil to said aluminum pad with electrically conductive glue; attaching, sputtering, plating, ion implanting or ionbeam-assisted depositing (IBAD) a titanium ring on to the platinum or iridium foil; adhering an insulating layer to the titanium ring, where insulation material is selected from the group consisting of silicon carbide, diamond-like coating, silicon nitride and silicon oxide in combination, titanium oxide, tantalum oxide, aluminum nitride, aluminum oxide and zirconium oxide.
266. The method as in paragraph 264 or paragraph 265 further comprising the step of plating an aluminum layer onto the exposed part of the titanium ring and onto a material selected from a group consisting of silicon carbide, diamond-like coating, silicon nitride and silicon oxide in combination, titanium oxide, tantalum oxide, aluminum nitride, aluminum oxide and zirconium oxide, said aluminum layer acting as an electrical conductor.
267. The method as in paragraph 265 further comprising the steps of forming said platinum electrode internal to a well formed by a substance selected from the group consisting of silicon carbide, diamond-like coating, silicon nitride and silicon oxide in combination, titanium oxide, tantalum oxide, aluminum nitride, aluminum oxide, zirconium oxide, PTFE (polytetrafluoroethylene), FEP (fluorinated ethylene propylene and waxes, and its titanium ring; fabricating said electrode with its whole peak angle in the range from1 to 120 ; forming the base of said conical or pyramidal electrode in the range from1 J. m to 500pm; forming the linear section of the well unoccupied by said conical or pyramidal electrode in the range ranging from zero to one-third of the whole linear section.
268. The method as in paragraph 265 further comprising the steps of forming the platinum electrode with a mushroom-like shape with a narrower stalk; constructing said electrode head with a larger plan view area than the well from which it arises.

## Claims

**1.** A visual prosthesis comprising:
an image receiver for receiving an image and converting said image to a signal;
a data processing unit, coupled to said image receiver and processing said signal;
a patient's controller within a housing suitable to place external to a human body;
a manually operable user interface on said housing, accepting user input and controlling said data processing unit and, thereby, electrode signal parameters; and
a plurality of electrodes driven in accordance with said signal and said electrode signal parameters, stimulating visual neurons to create a perception of said image.

**2.** The visual prosthesis according to claim 1, wherein said user interface controls frequency of a signal on said electrodes.

**3.** The visual prosthesis according to claims 1 or 2, wherein said user interface controls amplitude of a signal on said electrodes.

**4.** The visual prosthesis according to any of claims 1 - 3, wherein said user interface controls pulse width of a signal on said electrodes.

**5.** The visual prosthesis according to claims 1-4, wherein said user interface controls brightness.

**6.** The visual prosthesis according to any of claims 1 - 5, wherein said user interface controls contrast.

**7.** The visual prosthesis according to any of claim 1-6, wherein said user interface controls zoom.

**8.** The visual prosthesis according to any of claims 1 - 7, wherein said user interface controls stimulation parameter of said plurality of electrodes individually.

**9.** The visual prosthesis according to any of claim 1 - 8, wherein said user interface and said patient's controller include telecommunications means wherein said user interface can be operated remotely from patient controller.

**10.** The visual prosthesis according to any of claim 1 - 9, wherein said user interface controls patterns of stimulation of said plurality of electrodes.

**11.** The visual prosthesis according to claim 10, wherein said patterns of stimulation include multipolar field focusing patterns of stimulation.

**12.** The visual prosthesis according to claim 11, wherein said field focusing patterns of stimulation include two or more electrodes.

**14.** The visual prosthesis according to any of claims 1 - 12, further comprising an internal communication unit which transmits operational data to said patient controller.
